# EUROPEAN PATENT APPLICATION

(11) **EP 4 424 694 A1**
(43) Date of publication of application: **04.09.2024**
(21) Application number: 22885962.5
(22) Date of filing: 25.10.2022
(51) Int. Cl.: C07H 15/00, C07H 1/00, A61P 25/00, A61P 25/28, A61K 31/35

(54) **ISOPROPYL-D-GLUCOPYRANOSIDE DERIVATIVE, AND PREPARATION METHOD THEREFOR AND USE THEREOF**

(30) Priority: 25.10.2021 US 202163271411 P
(71) Applicant: Chen, Linyi, Hsinchu, Taiwan 30013 (CN)
(72) Inventor: WANG, Yi, Hsinchu, Taiwan 30013 (TW); LIAO, Wen-Ling, Hsinchu, Taiwan 30013 (TW); LEE, Yu-Tang, Hsinchu, Taiwan 30013 (TW); LU, Ting-Hsuan, Hsinchu, Taiwan 30013 (TW); HUANG, Yu-Wen, Hsinchu, Taiwan 30013 (TW); LI, Chia-Wei, Hsinchu, Taiwan 30013 (TW); WANG, Chen, Hsinchu, Taiwan 30013 (TW); CHEN, Fang-Yi, Hsinchu, Taiwan 30013 (TW); CHIAO, Chuan-Chin, Hsinchu, Taiwan 30013 (TW); CHEN, Linyi, Hsinchu, Taiwan 30013 (TW)
(74) Representative: karo IP
(86) International application number: PCT/CN2022/127414
(87) International publication number: WO 2023/072085

(57) **Abstract**

The invention relates to a new chemical compound of Isopropyl-D-glucopyranoside derivatives and its chemical synthesis to prepare the chemical compound. The invention also provides uses of the Isopropyl-D-glucopyranoside derivatives for promoting regeneration of injured brain neurons and retinal neurons.

## Description

### CROSS-REFERENCE TO RELATED APPLICATIONS

This application is priority under 35 U.S.C. § 119(e) to U.S. Provisional Patent Application No.: 63/271,411, filed on Oct. 25, 2021, PCT International Application No. PCT/CN2022/127414, filed Oct. 25, 2022, and Taiwan Patent Application No. 111140500, filed on Oct. 25, 2022, which is incorporated by reference in its entirety herein.

### FIELD OF THE INVENTION

This disclosure relates to an Isopropyl-D-glucopyranoside derivative, its synthesis method and use of the derivative of isopropyl-D-glucopyranoside for promoting nerve repair.

### BACKGROUND OF THE INVENTION

Current nerve injury, such as Traumatic Brain Injury (TBI), affect approximately seventy million people globally each year. Common treatment modalities include physical therapy, hyperbaric oxygen therapy, transcranial magnetic stimulation, and transcranial direct current stimulation. These non-invasive therapies can improve depression and cognitive function post-TBI, yet there is currently no effective medication to promote neural regeneration following brain injury.

Traumatic Brain Injury (TBI) is damage to the brain caused by external force or impact. There are approximately nearly 70 million confirmed cases globally each year. TBI can impair brain nerves, leading to deficits in motor or cognitive functions in patients. Due to the difficulty of central nervous system regeneration and recovery after damage, there are currently no effective therapies in medicine to promote neural regeneration for TBI. Patients with traumatic brain injuries often develop brain lesions over time, which may lead to degenerative neurological diseases in the future. Therefore, early administration of medications that promote neural regeneration following brain injury is the solution for treatment.

Furthermore, in terms of selecting therapeutic drugs, due to the presence of the blood-brain barrier, conventional medications cannot easily penetrate the blood-brain barrier to reach effective concentrations. Therefore, when treating brain disorders, the administration of effective medications becomes a significant challenge.

Given this, there is an urgent need to develop a medication for treating nerve injury. Additionally, there is a critical need for a drug delivery method capable of penetrating the blood-brain barrier.

### SUMMARY OF THE INVENTION

The human nervous system comprises the central nervous system (CNS) and the peripheral nervous system (PNS), both composed of neurons. The trigeminal nerve is a cranial nerve within the peripheral nervous system that connects to the brainstem (central nervous system).

Therefore, when the Isopropyl-d-glucopyranoside derivative, as described in present invention, traverse the nasal mucosa, pass through the olfactory epithelial cells, and enter the pathway surrounding the olfactory and trigeminal nerves, they can reach the brain. Simultaneously, they can also reach the peripheral nervous system, thereby achieving a systemic effect in neural repair.

Given this, in present invention, the experiment focuses on using the less reparable cranial nerves. This approach aims to achieve a reparative effect on both the central nervous system and the peripheral nervous system within the neural system.

The "central nervous system" comprises the brain and spinal cord. In the context of this invention, the term "central nervous system" includes, but is not limited to, the olfactory bulb, amygdala, hippocampus, neocortex, lateral ventricles, superior colliculus, thalamus, hypothalamus, pituitary gland, pineal gland, third ventricle, midbrain tectum, cerebral peduncle, frontal lobe, cerebral aqueduct, brainstem, cerebellum, and spinal cord.

The "peripheral nervous system" is composed of the somatic nervous system and the autonomic nervous system. In the context of this invention, the term "peripheral nervous system" includes, but is not limited to, sensory nerves, motor nerves, cranial nerves, spinal nerves, sympathetic nerves, parasympathetic nerves, and the enteric nervous system.

In view of the above-mentioned problem, the present invention provides a Isopropyl-D-glucopyranoside derivative, wherein the Isopropyl-D-glucopyranoside derivative has the structure of Formula (1), 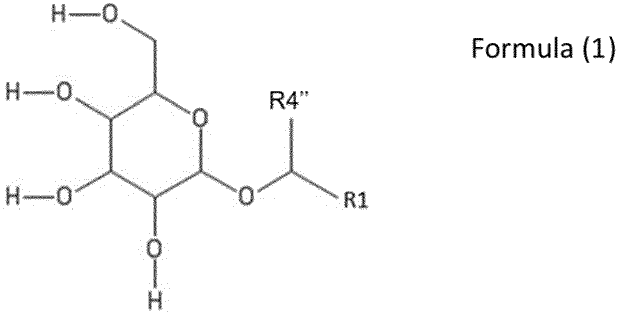 wherein R1 can is substituted or nonsubstituted Formula (2) or Formula (3), 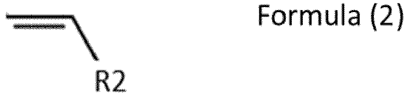 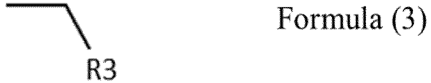 wherein R2 and R3 is substituted or nonsubstituted Formula (4) or Formula (5), 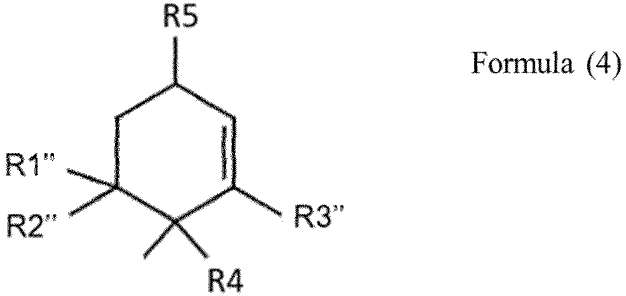 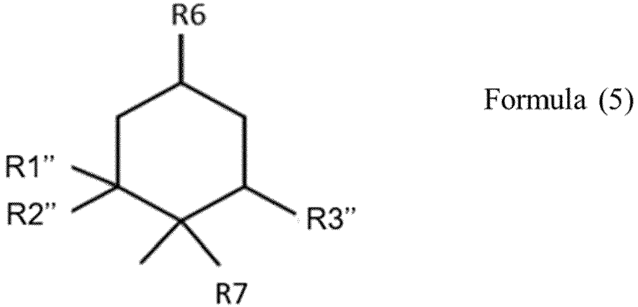
wherein R4 is hydrogen, deuterium, tritium, hydroxyl, or halogen,
wherein R5 is hydrogen, deuterium, tritium, hydroxyl, carbonyl, or halogen,
wherein R6 is hydrogen, deuterium, tritium, hydroxyl, carbonyl, or halogen,
wherein R7 is hydrogen, deuterium, tritium, hydroxyl, carbonyl, or halogen,
wherein R1" is Methyl, ethyl, propyl, hydrogen, deuterium, tritium, or hydroxyl,
wherein R2" is Methyl, ethyl, propyl, hydrogen, deuterium, tritium, or hydroxyl,
wherein R3" is Methyl, ethyl, propyl, hydrogen, deuterium, or tritium.,
wherein R4" is Methyl, ethyl, propyl, hydrogen, deuterium, or tritium.

In some embodiments, the Isopropyl-D-glucopyranoside derivative comprises following compounds: 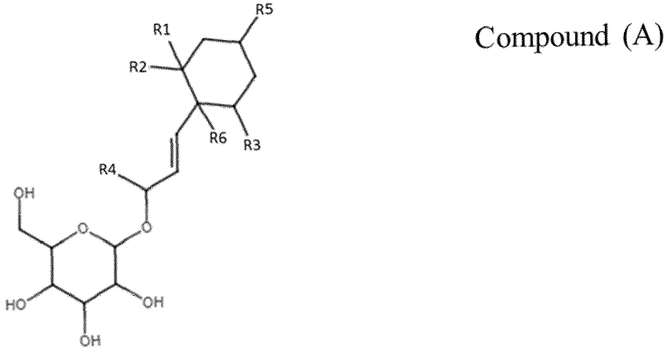 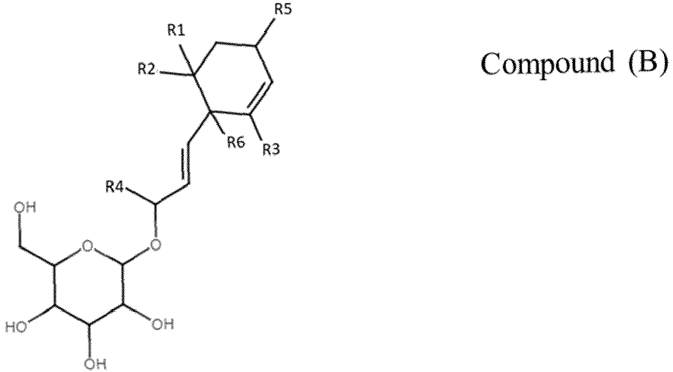 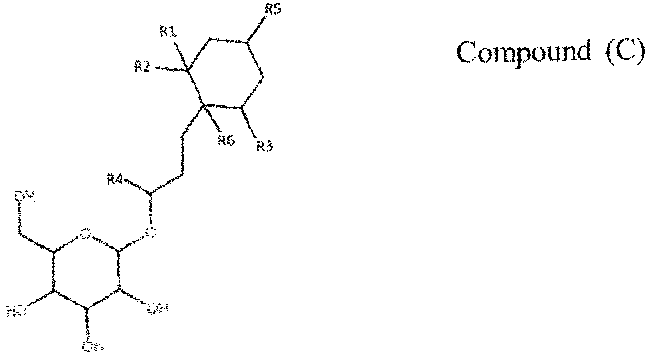 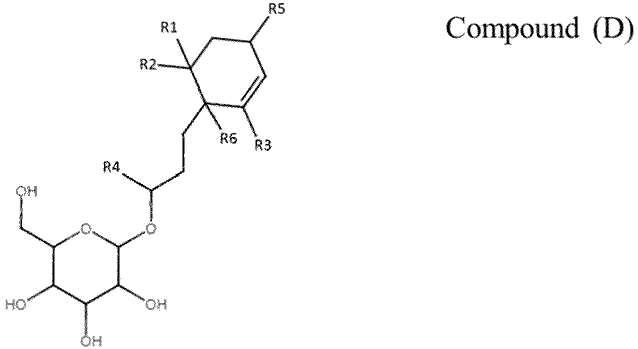
wherein R1 is methyl, ethyl, propyl, hydrogen, deuterium, tritium, or hydroxyl,
wherein R2 is methyl, ethyl, propyl, hydrogen, deuterium, tritium, or hydroxyl,
wherein R4 is methyl, ethyl, propyl, hydrogen, deuterium, or tritium,
wherein R4 is methyl, ethyl, propyl, hydrogen, deuterium, or tritium,
wherein R5 is hydrogen, deuterium, tritium, hydroxyl, carbonyl, or halogen,
wherein R6 is hydrogen, deuterium, tritium, hydroxyl, or halogen.

In present invention, the Isopropyl-D-glucopyranoside derivative is used for the treatment of nerve injuries. To enable those skilled in the art to understand the content of the invention claimed in the patent application, the present invention utilizes examples such as Ampelopsisionoside, Byzantionoside B, and Roseoside from the derivative of Isopropyl-D-glucopyranoside.

In some embodiments, the Isopropyl-D-glucopyranoside derivative of Formula (6) is prepared by following chemical synthesis steps: 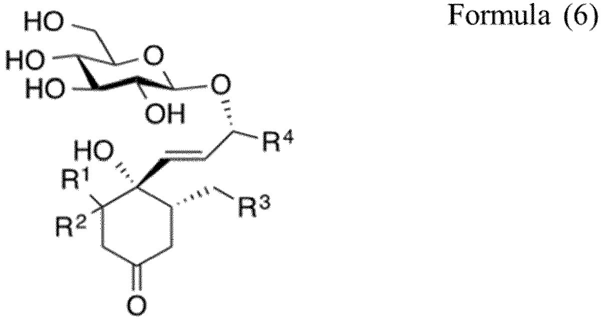
(A) As shown in the following Scheme 1, transforming Formula (7) into Formula (8) via including a dialkylation, an acylation, and an acetylide addition; 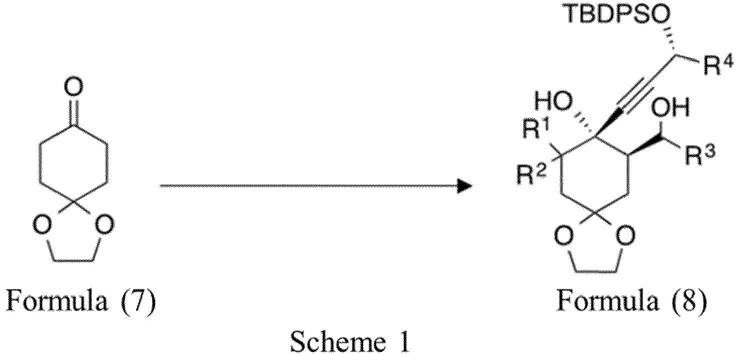
   wherein Rican be Me, Et, Pr, H, or OH; R2 can be Me, Et, Pr, H, or OH; R3 can be Me, Et, Pr, or H; R4 can be Me, Et, Pr or H;
   wherein the dialkylation is carried out with an electrophilic reagent 1, which can be MeI, EtI, MeOTf, EtOTf, PrI, PrBr/NaI, or Me₂SO₄;
   wherein in the acylation, the reagent can be HCHO, MeCHO, or EtCHO;
   wherein this intermediate undergoes further anionic nucleophilic addition reaction with an alkyne, leading to the formation of compound (8), wherein the alkyne nucleophilic addition reagent comprises an alkyne bearing a functional group R4, along with a base, which includes n-butyllithium (nBuLi), lithium diisopropylamide (LDA), or lithium bis(trimethylsilyl)amide (LHMDS), wherein the functional group R4 can be methyl, ethyl, propyl, hydrogen, deuterium, or tritium;
(B) As shown in the following Scheme 2, transforming compound of Formula (8) into compound of Formula (9) by an olefination, a reduction, and a deprotection; 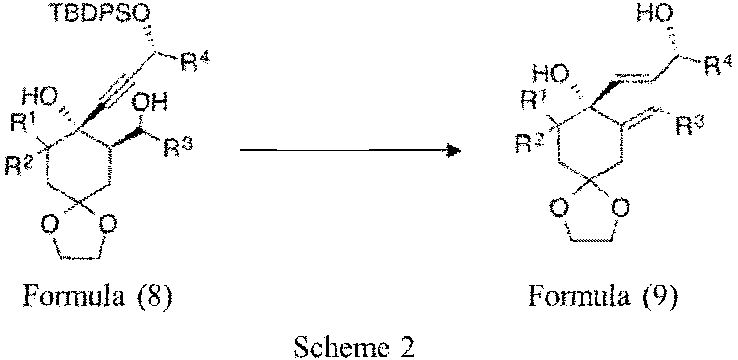
   wherein the compound of Formula (8) in the olefination is first performed with an electrophilic reagent 2 and an amine base reagent at a reduced temperature to form the compound of Formula (9), wherein the electrophilic reagent 2 can be mesyl chloride, tosyl chloride, acetic anhydride, benzoic anhydride, methyl iodide, and dimethyl sulfate, wherein the amine base reagent can be triethyl amine, diethyl amine, pyridine, pyrrolidine, ethyldisiopropyl amine, 2,6-lutidine, and 1,4-diazabicyclo[2,2,2]octane, wherein the reduced temperature is -30 to 25°C,
   after that a base 2 is then added to the above reaction mixture to complete the olefination, wherein the base 2 can be potassium tert-butoxide, sodium hydroxide, sodium methoxide, or 1,8-diazabicyclo[5,4,0]undec-7-ene,
   following completion, the reduction reaction is carried out with a reducing agent 1, wherein the reducing agent 1 can be LiAlH₄, diisobutyl aluminum hydride (DIBAL), NaBH₄, NaB(OAc)₃H, Lithium triethylborohydride, or sodium bis(2-methoxyethoxy)luminiumhydride, the reaction temperature of the reduction is -50 to 25°C;
   wherein following completion, the deprotection is carried out with a desilylation reagent 1 to conduct the deprotection reaction to form formula (9), wherein the deprotection reagent 1 is tetrabutyl ammonium fluoride (TBAF), HF-py, HCl, or potassium tert-butoxide, wherein the reaction temperature of the deprotection is -30 to 25 °C;
(C) As shown in the following Scheme 3, Reacting Formula (9) with Formula (10) by glycosylation to form Formula (11); 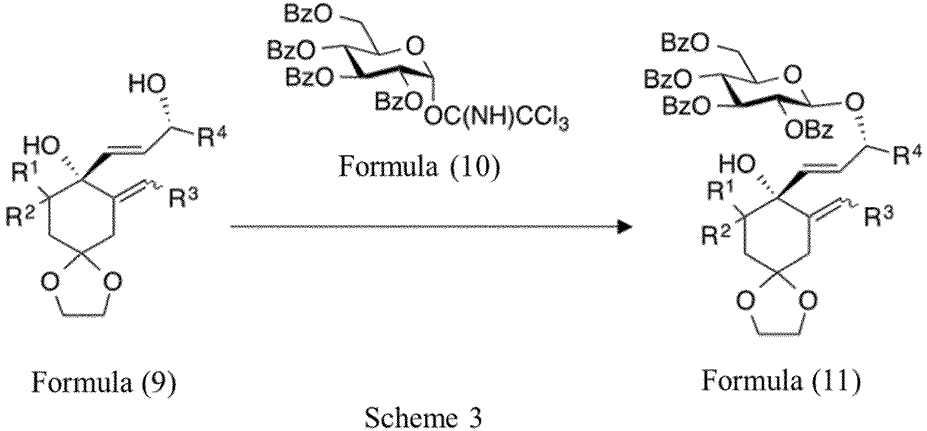 wherein the compound of Formula (9) undergoes the glycosylation reaction carried out with a glycosylating agent 1 to yield the compound of Formula (10), wherein the glycosylation reagent 1 can be NIS/AgOTf, BSP/TTBP/ Tf2O, TMSOTf/NIS, TMSOTf, AgOTf, or CuOTf, wherein the reaction temperature of the deprotection is -78 to 0 °C;
(D) Transforming the compound of Formula (11) into the compound of Formula (6) by including a deprotection of acetal, an isomerization, a removal of benzoyl group protection, and a 1,4-reduction;
   wherein the compound of Formula (11) undergoes the deprotection of acetal and the isomerization initially, wherein the deprotection of acetal and the isomerization are carried out with a reagent 1, wherein the reagent 1 can be AcOH(aq), HCl(aq), TFA+H₂O, TsOH+H₂O, HCl in 1,4-dioxane, or H₂SO₄(aq);
   subsequently, removal of benzoyl group is carried out using a deprotection reagent, wherein the deprotection reagent can be NaOH, NaOMe, NaOEt, KOH, KOMe, KOEt, HCl in MeOH, HCl(aq), TFA+ H₂O, or H₂SO₄ (aq);
   following that, a 1,4-reduction is conducted to obtain the compound of Formula (6), where the 1,4-reduction is carried out with a 1,4-reduction reagent, wherein the 1,4-reduction reagent can be NaBH₄, LiAlH₄, NaB(OAc)₃H, NaBCNH₃, L-selectride, [CuH(PPh₃)]₆, diisobutyl aluminum hydride (DIBAL), or H₂/Pd/C, wherein the reaction temperature of the 1,4-reduction is -78 to 25 °C;
   wherein R1 is methyl, ethyl, propyl, hydrogen, deuterium, tritium, or hydroxyl,
   wherein R2 is methyl, ethyl, propyl, hydrogen, deuterium, tritium, or hydroxyl,
   wherein R4 is methyl, ethyl, propyl, hydrogen, deuterium, or tritium,
   wherein R4 is methyl, ethyl, propyl, hydrogen, deuterium, or tritium.

In some embodiments, the Isopropyl-d-glucopyranoside derivative compound of Formula (12) is synthesized by the following chemical synthesis steps: 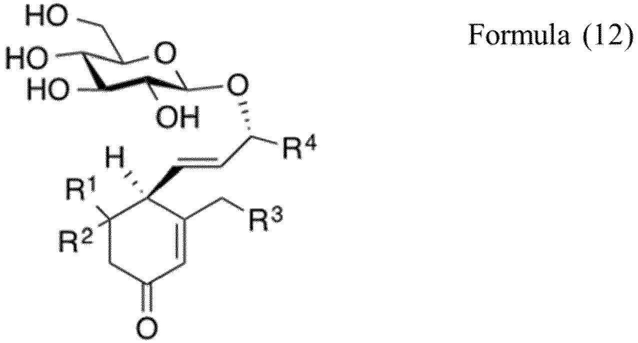
(A) As shown in the following Scheme 4, transforming the compound of Formula (11) into the compounds of Formulas (12); 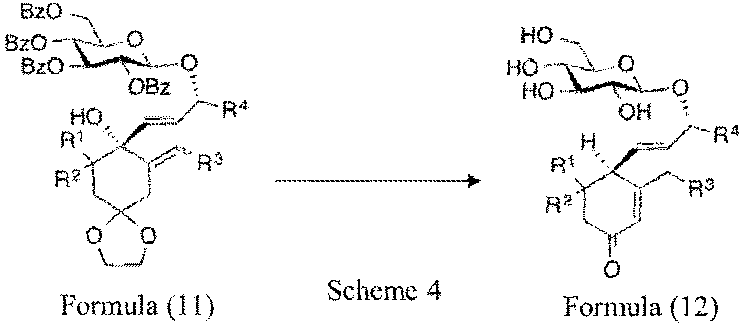
   wherein the conversion reaction comprises a deprotection of acetal, an isomerization, a stereoselective deoxygenation, and a removal of benzoyl protection, wherein the deprotection of acetal and the isomerization is carried out with an acidic reagent, wherein the acidic reagent can be AcOH(aq), HCl(aq), TFA+H₂O, TsOH+H₂O, HCl in 1,4-dioxane, or H₂SO₄(aq);
   following by a stereoselective deoxygenationn with a reducing reagent 2 at a reduced temperature 1, wherein the reducing reagent 2 can be NaBH₄, LiAlH₄, NaB(OAc)₃H, NaBCNH₃, Et₃SiH/BF₃-Et₂O, L-selectride, [CuH(PPh₃)]₆, or diisobutyl aluminum hydride (DIBAL), wherein the amount of the reducing reagent can be 0.5, 1.0, 2.0, 3.0, 4.0, or 5.0 equivalences, wherein the reduced temperature 1 in the stereoselective deoxygenation is -78 to 25 °C;
   after that the intermediate is then subjected to removal of benzoyl group with a deprotection reagent 2 to produce the compound of the Formula (11), wherein the deprotection reagent 2 can be NaOH, NaOMe, NaOEt, KOH, KOMe, KOEt, HCl in MeOH, HCl(aq), TFA+H₂O, or H₂SO₄(aq);
   wherein R1 is methyl, ethyl, propyl, hydrogen, deuterium, tritium, or hydroxyl,
   wherein R2 is methyl, ethyl, propyl, hydrogen, deuterium, tritium, or hydroxyl,
   wherein R4 is methyl, ethyl, propyl, hydrogen, deuterium, or tritium,
   wherein R4 is methyl, ethyl, propyl, hydrogen, deuterium, or tritium.

In some embodiments, the Isopropyl-d-glucopyranoside derivative compound of Formula (13) is synthesized by the following chemical synthesis steps: 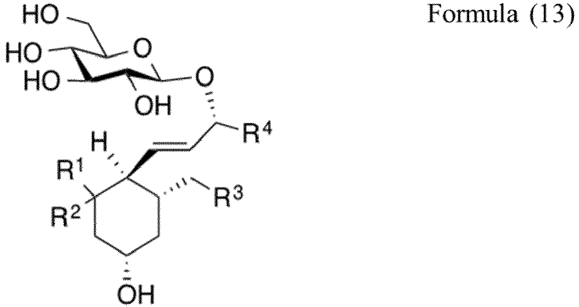
(A)As shown in the following Scheme 5, transforming the compound of Formula (12) into the compounds of Formulas (13) 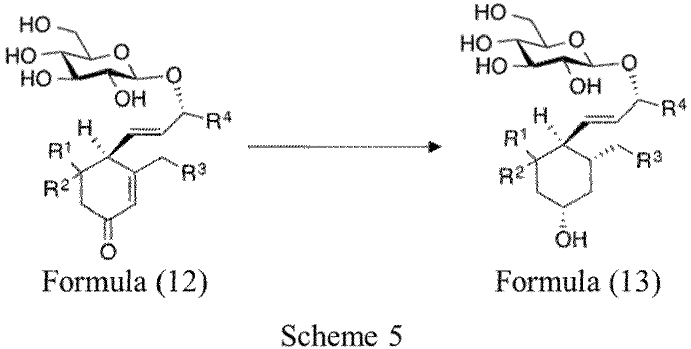
   wherein the conversion reaction comprises a regio- and stereo-selective 1,4-reduction/carbonyl reduction; wherein the regio- and stereo-selective 1,4-reduction is performed with a reducing reagent 3 at a reduced temperature 2, wherein the reducing reagent 3 can be NaBH₄, LiAlH₄, NaB(OAc)₃H, NaBCNH₃, L-selectride, [CuH(PPh₃)]₆, diisobutyl aluminum hydride (DIBAL) or H₂/Pd/C, wherein the amount of the reducing reagent 3 can be 0.5, 1.0, 2.0, 3.0, 4.0, or 5.0 equivalences, wherein the reduced temperature 2 in the regio- and stereo-selective 1,4-reduction/carbonyl reduction is -78 to 25 °C;
   wherein R1 is methyl, ethyl, propyl, hydrogen, deuterium, tritium, or hydroxyl,
   wherein R2 is methyl, ethyl, propyl, hydrogen, deuterium, tritium, or hydroxyl,
   wherein R4 is methyl, ethyl, propyl, hydrogen, deuterium, or tritium,
   wherein R4 is methyl, ethyl, propyl, hydrogen, deuterium, or tritium.

In some embodiments, the Isopropyl-d-glucopyranoside derivative compound of Formula (14) is synthesized by the following chemical synthesis steps: 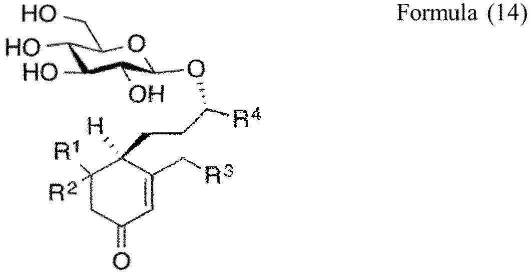
(A) As shown in the following Scheme 6, transforming the compound of Formula (12) into the compound of Formula (14) through a selective alkene reduction; 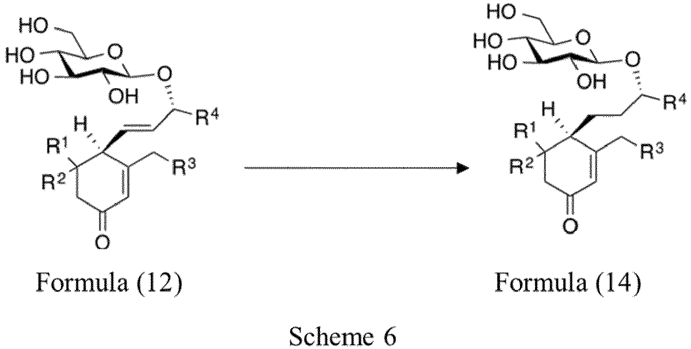
   wherein the selective alkene reduction is carried out with a reducing reagent 4, wherein the reducing reagent 4 can be NaBH₄, LiAlH₄, NaB(OAc)₃H, NaBCNH₃, L-selectride, [CuH(PPh₃)]₆, diisobutyl aluminum hydride (DIBAL), H₂/Pd/C or RhCl(PPH₃)₃/H₂, wherein the reduced temperature in the selective alkene reduction is -78 to 25 °C;
   wherein R1 is methyl, ethyl, propyl, hydrogen, deuterium, tritium, or hydroxyl,
   wherein R2 is methyl, ethyl, propyl, hydrogen, deuterium, tritium, or hydroxyl,
   wherein R4 is methyl, ethyl, propyl, hydrogen, deuterium, or tritium,
   wherein R4 is methyl, ethyl, propyl, hydrogen, deuterium, or tritium.

In some embodiments, the Isopropyl-d-glucopyranoside derivative compound of Formula (15) is synthesized by the following chemical synthesis steps: 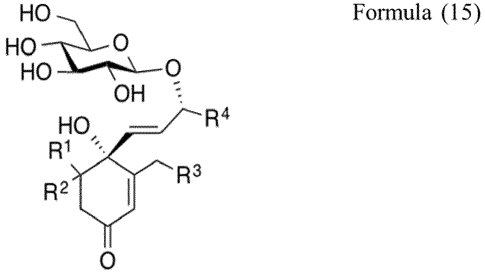
(A) As shown in the following Scheme 7, converting the compound of Formula (9) by a deprotection of acetal, an isomerization, and a removal of benzoyl group to yield the compound of Formula (15); 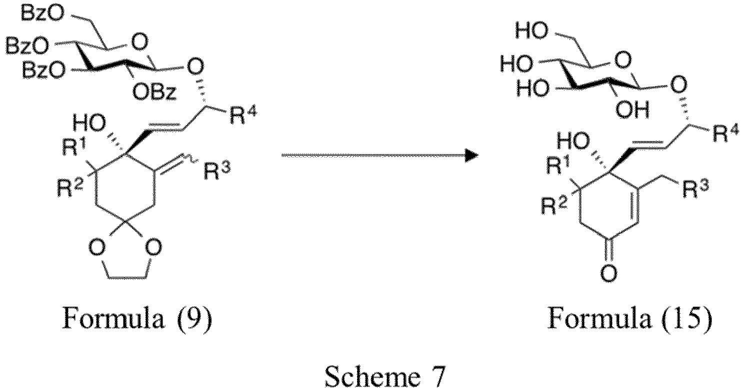
   wherein the compound of Formula (9) undergoes the deprotection of acetal and the isomerization initially, wherein the deprotection of acetal and the isomerization are carried out with a deprotection of acetal and isomerization reagent, respectively, wherein the deprotection of acetal and isomerization reagent can be AcOH(aq), HCl(aq), TFA+H₂O, TsOH+H₂O, HCl in 1,4-dioxane, or H₂SO₄(aq);
   subsequently, removal of benzoyl group is carried out using a deprotection reagent, wherein the deprotection reagent can be NaOH, NaOMe, NaOEt, KOH, KOMe, KOEt, HCl in MeOH, HCl(aq), TFA+ H₂O, or H₂SO₄ (aq);
   wherein R1 is methyl, ethyl, propyl, hydrogen, deuterium, tritium, or hydroxyl,
   wherein R2 is methyl, ethyl, propyl, hydrogen, deuterium, tritium, or hydroxyl,
   wherein R4 is methyl, ethyl, propyl, hydrogen, deuterium, or tritium,
   wherein R4 is methyl, ethyl, propyl, hydrogen, deuterium, or tritium.

In some embodiments, the Isopropyl-d-glucopyranoside derivative compound of Formula (16) and Formula (17) is synthesized by the following chemical synthesis steps: 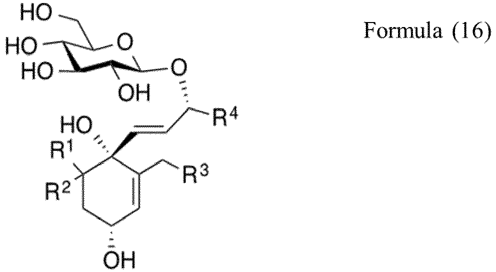 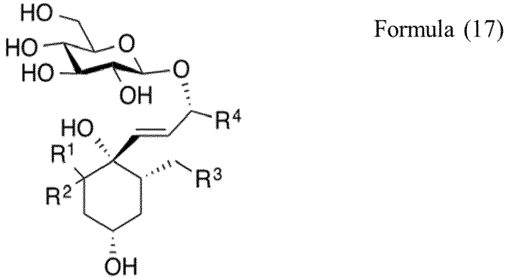 As shown in the following Scheme 8, transforming the compound of Formula (15) into the compounds of Formulas (16) and Formulas (17) by a selective reduction reaction; 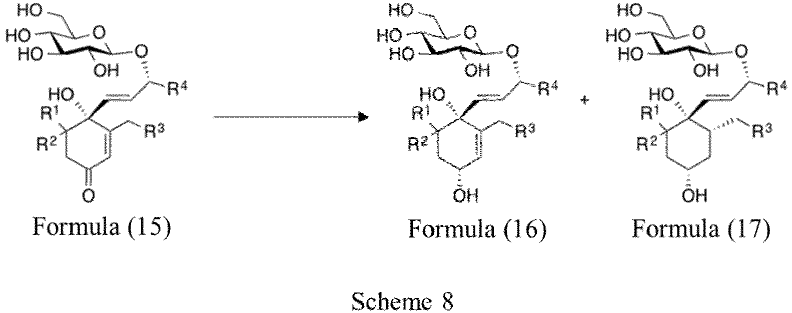
wherein the compound of Formula (15) is subjected to selective reduction carried out with a selective reduction reagent to obtain the compounds of formulas (16) and (17), wherein the selective reduction reagent can be NaBH₄, LiAlH₄, NaB(OAc)₃H, NaBCNH₃, L-selectride, [CuH(PPh₃)]₆, diisobutyl aluminum hydride (DIBAL) or H₂/Pd/C, wherein the reaction temperature of the selective reduction is -78 to 25 °C;
wherein R1 is methyl, ethyl, propyl, hydrogen, deuterium, tritium, or hydroxyl,
wherein R2 is methyl, ethyl, propyl, hydrogen, deuterium, tritium, or hydroxyl,
wherein R4 is methyl, ethyl, propyl, hydrogen, deuterium, or tritium,
wherein R4 is methyl, ethyl, propyl, hydrogen, deuterium, or tritium.

In some embodiments, the Isopropyl-d-glucopyranoside derivatives exhibits low toxicity towards Neuro2a cells.

The present invention further provides a use of the Isopropyl-d-glucopyranoside derivatives for promoting neural repair.

In some embodiments, the Isopropyl-d-glucopyranoside derivatives, when used alone, can promote the regeneration of injured hippocampal neurons, and facilitate neural regeneration in 3D brain tissue slices. This demonstrates the effectiveness of the compound in promoting neuronal regeneration and its potential use in repairing nerve injury.

In some embodiments, the Isopropyl-d-glucopyranoside derivatives can promote the repair of retinal nerves.

In some embodiments, the Isopropyl-d-glucopyranoside derivatives can penetrate the blood-brain barrier of the user, thereby entering the brain to facilitate the repair of neurons injury.

In some embodiments, the Isopropyl-d-glucopyranoside derivatives can penetrate the blood-brain barrier, enabling them to enter the brain and promote the repair, regeneration, or increase in the number of brain neurons.

In some embodiments, the Isopropyl-d-glucopyranoside derivatives can promote the regeneration of injured cortical neurons.

In some embodiments, the effective dose range for Isopropyl-d-glucopyranoside derivatives is from 9.674 nM to 1342 µM.

In some embodiments, the Isopropyl-d-glucopyranoside derivatives have a significant effect on neural repair. When administered nasally, these compounds can penetrate the blood-brain barrier, facilitating the repair of damaged neurons. They promote neural repair, regeneration, or an increase in the number of neurons in the brain. Additionally, they facilitate nerve axon regeneration in injured cortical neurons and hippocampal neurons.

It is to be understood that both the foregoing general description and the following detailed description are exemplary and explanatory only and are not restrictive of the invention.

### BRIEF DESCRIPTION OF THE DRAWINGS

**Fig. 1-1C** show that the cytotoxicity experimental results of Isopropyl-D-glucopyranoside derivatives in present invention
**Fig. 2** shows that the flowchart of *in vitro* neural repair assay in present invention.
**Fig. 3** shows that the reference schematic diagram for calculating gap closure rate in the present invention.
**Figs. 4A-4B** show that the results of the *in vitro* hippocampal neuronal repair experiments involving Isopropyl-D-glucopyranoside derivative Ampelopsisionoside in the present invention.
**Fig. 5** shows that the results of the *in vitro* cortical neuronal regeneration experiments involving the Isopropyl-D-glucopyranoside derivative Ampelopsisionoside derivative Narirutin in the present invention.
**Fig. 6** shows that the results of the *in vitro* hippocampal neuronal repair experiments involving Isopropyl-D-glucopyranoside derivative Byzantionoside B in the present invention.
**Fig. 7** shows that the results of the *in vitro* cortical neuronal regeneration experiments involving the Isopropyl-D-glucopyranoside derivative Byzantionoside B derivative Narirutin in the present invention.
**Fig. 8** shows that the results of the *in vitro* hippocampal neuronal repair experiments involving Isopropyl-D-glucopyranoside derivative Roseoside in the present invention.
**Fig. 9** shows that the results of the *in vitro* cortical neuronal regeneration experiments involving the Isopropyl-D-glucopyranoside derivative Roseoside derivative Narirutin in the present invention.
**Fig. 10** shows that the flowchart *of ex vivo* brain tissue slice experiments in the present invention.
**Fig. 11** shows that the results of the *ex vivo* brain tissue slice experiments involving the Isopropyl-D-glucopyranoside derivative Ampelopsisionoside in the present invention.
**Fig. 12** shows that the results of *ex vivo* brain tissue slice experiments involving the Isopropyl-D-glucopyranoside derivative Byzantionoside B in the present invention.
**Fig. 13** shows that the results of *ex vivo* brain tissue slice experiments involving the Isopropyl-D-glucopyranoside derivative Roseoside in the present invention.
**Fig. 14** shows that the experimental flowchart for the Controlled Cortical Impact (CCI) model in the present invention, and the results of experiments promoting the recovery of motor function in mice after brain injury by Isopropyl-D-glucopyranoside derivative Ampelopsisionoside compound.
**Fig. 15** shows that the experimental flowchart for the Controlled Cortical Impact (CCI) model in the present invention, and the results of experiments promoting the recovery of environmental exploration ability (stress test) in mice after brain injury.
**Fig. 16** shows that the experimental flowchart for the Controlled Cortical Impact (CCI) model of the Isopropyl-D-glucopyranoside derivative Ampelopsisionoside compound in the present invention.
**Fig. 17** shows that the results of the horizontal bar test of the Controlled Cortical Impact (CCI) model of the Isopropyl-D-glucopyranoside derivative Ampelopsisionoside compound.
**Fig. 18** shows that the *ex vivo* experimental flowchart for of the Isopropyl-D-glucopyranoside derivative Byzantionoside B compound promoting retinal nerve repair in present invention.
**Fig. 19** shows that the *ex vivo* experimental results of Isopropyl-D-glucopyranoside derivative Byzantionoside B promoting retinal nerve repair in present invention.
**Fig. 20** shows that the *ex vivo* experimental flowchart for of the Isopropyl-D-glucopyranoside derivative Roseoside compound promoting retinal nerve repair in present invention
**Fig. 21** shows that the *ex vivo* experimental results of Isopropyl-D-glucopyranoside derivative Roseoside promoting retinal nerve repair in present invention

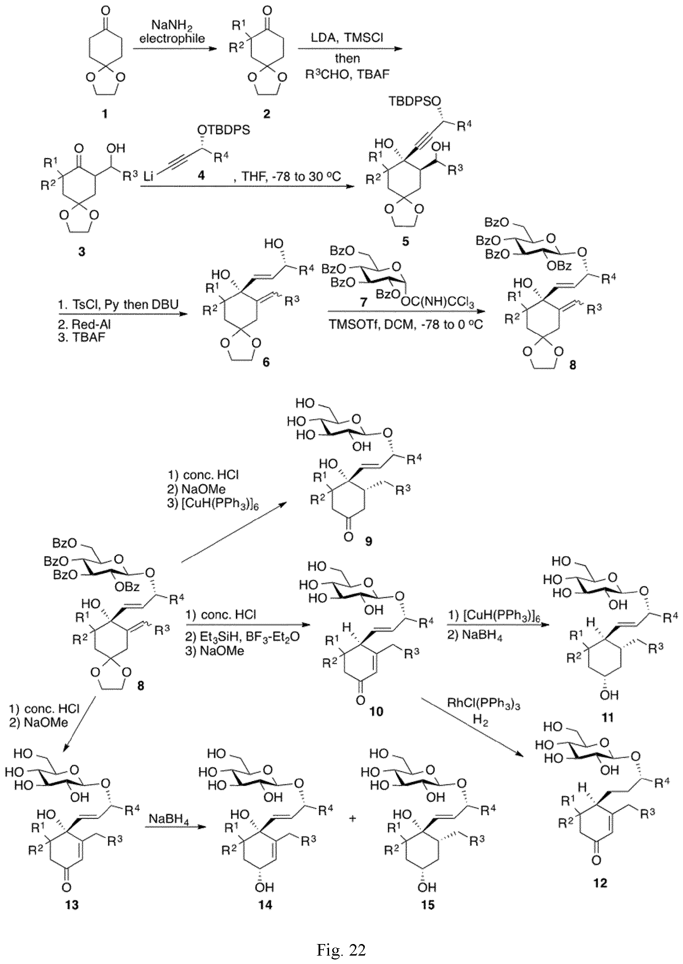

**Fig. 22** shows that the synthesis flowchart of Isopropyl-D-glucopyranoside derivatives in present invention

### DETAILED DESCRIPTION OF THE INVENTION

Additional specific embodiments of the present invention include, but are not limited to the following:

### EXAMPLE 1

### The cytotoxicity experiment of Isopropyl-D-glucopyranoside derivatives

To examine the toxicity profile of Ampelopsisionoside, Byzantionoside B, and Roseoside to neuro2a cells, we used CellTiter-Glo cell viability assay to perform the experiment. CellTiter-Glo cell viability assay can detect the level of ATP in neuro2a cells after treatment of compounds to evaluate the survival rate.

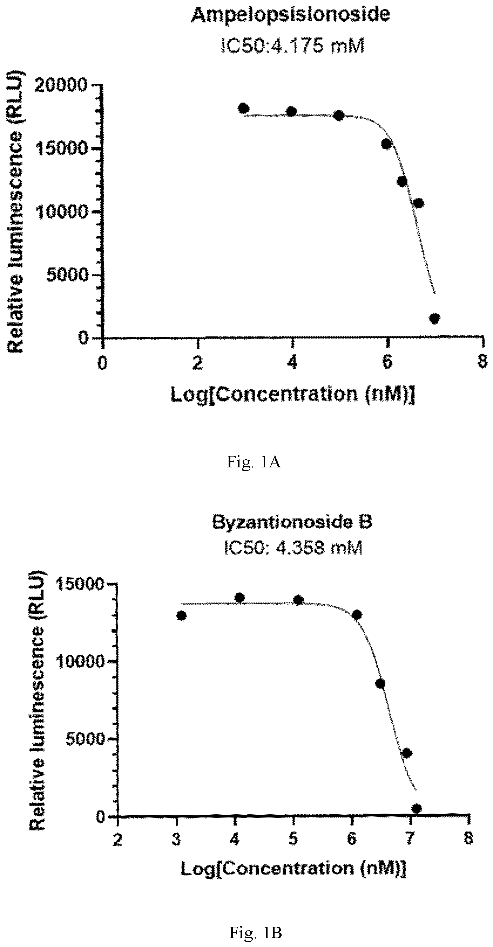

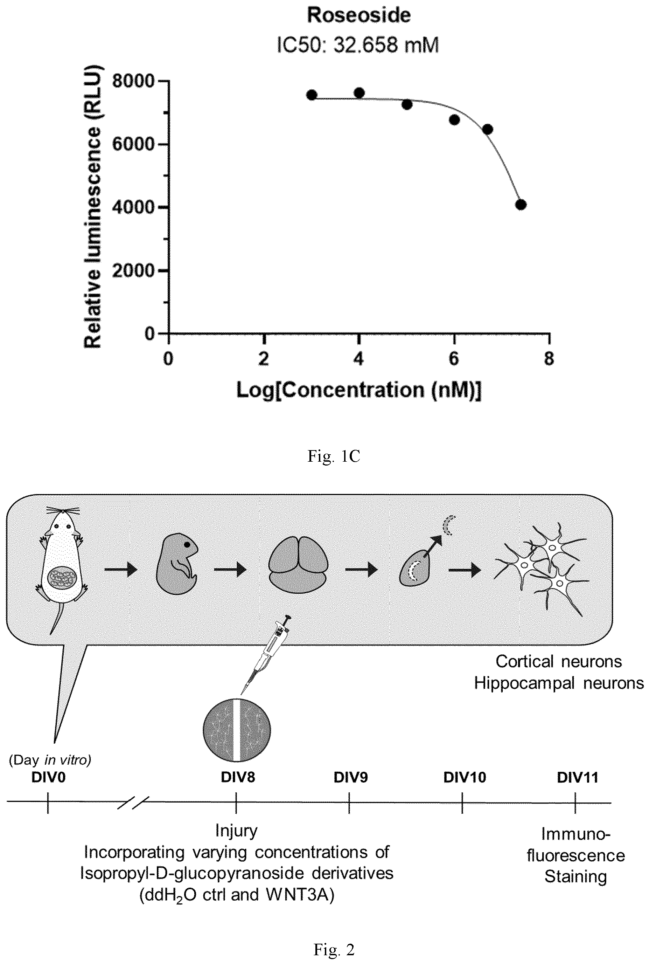

As shown in Figure 1A to 1C, the results shows that the IC50 of Ampelopsisionoside, Byzantionoside B, and Roseoside ranging from 4.175 mM to 32.658 mM, which suggest a good safety profiles of these compounds.

### EXAMPLE 2

### In vitro hippocampal neuron repair experiment of Isopropyl-D-glucopyranoside derivative Ampelopsisionoside

The experimental procedure is depicted in Figure 2. Removed the brain of E18 rat embryos and dissected into cortex and hippocampus. Subsequently, the cortex and hippocampus were separated into cortical neurons and hippocampal neurons, respectively. Then, seeded hippocampal neurons on 48-well plates. The first day that neurons are cultured *in vitro* is defined as day *in vitro* 0 (DIVO). On DIV2, Cytosine beta-D-arabinoside (AraC) was added to neurons to inhibit the proliferation of glial cells. On DIV8, neurons were injured using pipette tips (p10 tips) and 9.674 nM to 967.4 µM Ampelopsisionoside was added. 0.1% DMSO served as the solvent control group, and the results in the figures were standardized against the 0.1% DMSO group. After 72 hours, immunostaining was performed. TUJ1 antibody was used to label neuronal cells to observe the regrowth of neurites. Images were taken using Observer Z1 microscope.

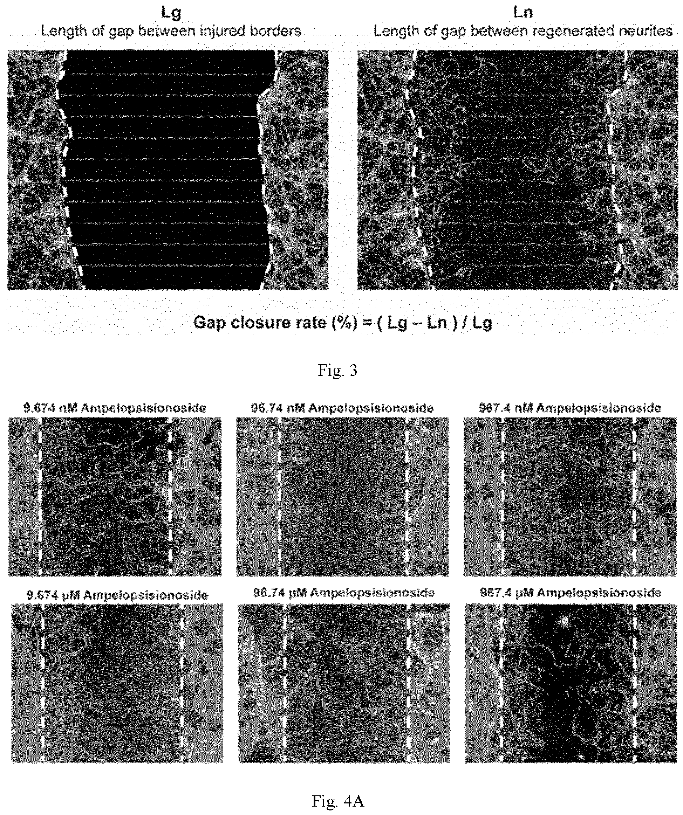

As depicted in Figure 3, the degree of neuronal axon regeneration was quantified using the gap closure rate. White dashed lines indicate the borders of injury gap, the central black area represents the region scratched by the tip of a pipette tips. The newly regenerated neurites grow from the white dashed lines toward the center of the gap. To quantify the results, we measured the lengths of gap (Lg) and distance between regenerated neurites (Ln) every 50 µm. After unbiasedly drawing ten lines of equal distance, the average distance between neurites is taken: Length of gap between regenerated neurons (Ln). Gap closure rate is calculated using the formula: (Lg-Ln)/Lg. Scale bar = 100 µm. Images were taken using Observer Z1 microscope.

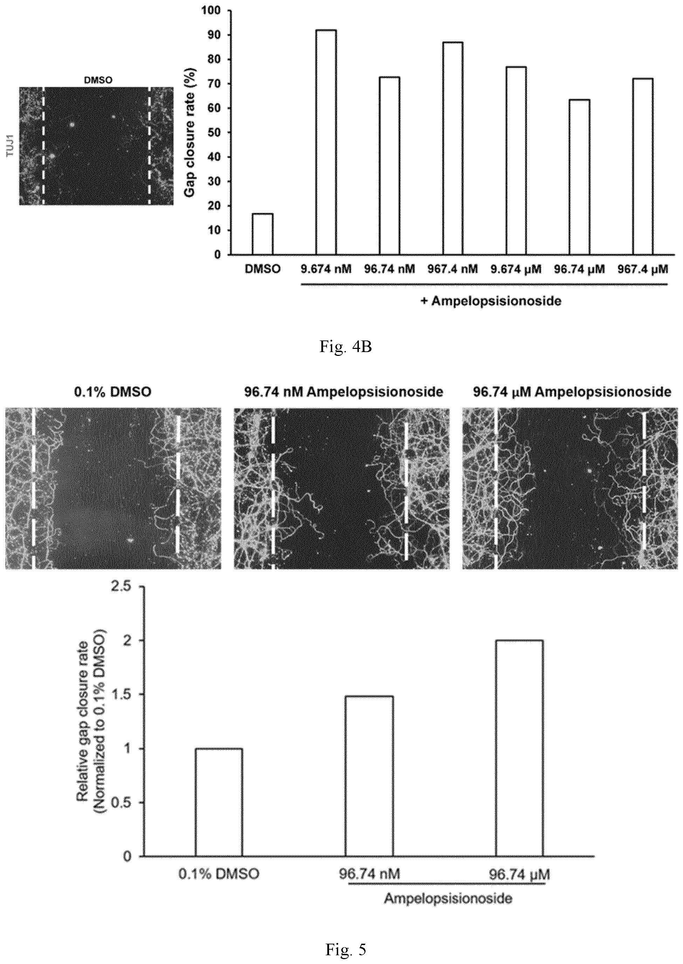

As shown in Figures 4A to 4B, quantifying the degree of axonal regeneration through the gap closure rate. The result indicates that Ampelopsisionoside promotes neurite regrowth after injury in hippocampal neurons.

### EXAMPLE 3

### In vitro cortical neuron repair experiment of Isopropyl-D-glucopyranoside derivative Ampelopsisionoside

Removed the brain of E18 rat embryos and dissected into cortex and hippocampus. Subsequently, the cortex and hippocampus were separated into cortical neurons and hippocampal neurons, respectively. Then, seeded cortical neurons on 48-well plates. The first day that neurons are cultured *in vitro* is defined as day *in vitro* 0 (DIVO). On DIV2, Cytosine beta-D-arabinoside (AraC) was added to neurons to inhibit the proliferation of glial cells. On DIV8, neurons were injured using pipette tips (p10 tips) and 9.674 nM to 96.74 µM Ampelopsisionoside was added. 0.1% DMSO served as the solvent control group, and the results in the figures were standardized against the 0.1% DMSO group. After 72 hours, immunostaining was performed. TUJ1 antibody was used to label neuronal cells to observe the regrowth of neurites. Scale bar = 100 µm. Images were taken using Observer Z1 microscope.

As shown in Figure 5, the degree of neuronal axon regeneration was quantified using the gap closure rate, the immunofluorescence image with two dashed lines representing the injury area. The results of this experiment indicate that Ampelopsisionoside effectively promotes dendritic outgrowth in cortical neurons.

### EXAMPLE 4

### In vitro hippocampal neuron repair experiment of Isopropyl-D-glucopyranoside derivative Byzantionoside B

Removed the brain of E18 rat embryos and dissected into cortex and hippocampus. Subsequently, the cortex and hippocampus were separated into cortical neurons and hippocampal neurons, respectively. Then, seeded hippocampal neurons on 48-well plates. The first day that neurons are cultured *in vitro* is defined as day *in vitro* 0 (DIVO). On DIV2, Cytosine beta-D-arabinoside (AraC) was added to neurons to inhibit the proliferation of glial cells. On DIV8, neurons were injured using pipette tips (p10 tips) and 0.13 µM to 1342 µM Byzantionoside B was added. ddH₂O served as the solvent control group, and the results in the figures were standardized against the 0.1% DMSO group. After 72 hours, immunostaining was performed. TUJ1 antibody was used to label neuronal cells to observe the regrowth of neurites. Scale bar = 100 µm. Images were taken using Observer Z1 microscope.

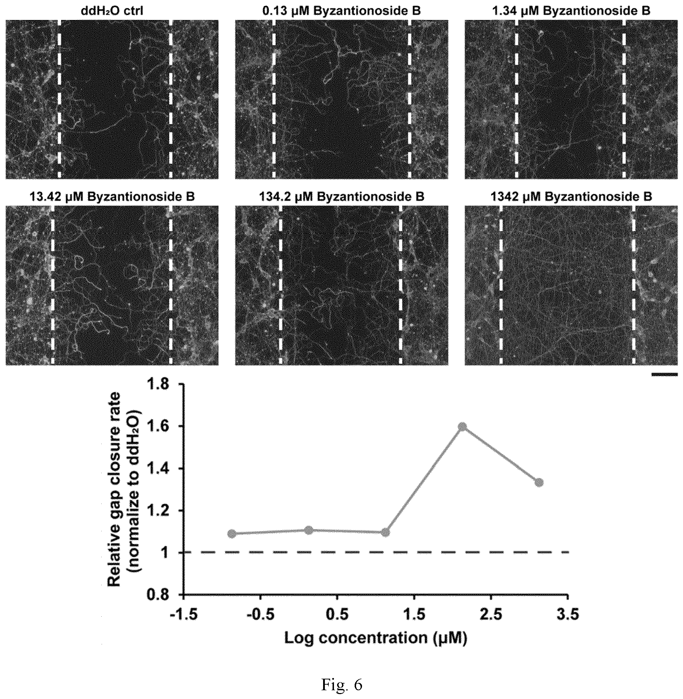

As shown in Figure 6, quantifying the degree of axonal regeneration through the gap closure rate, the immunofluorescence image with two dashed lines representing the injury area. The result indicates that Byzantionoside B promotes neurite regrowth after injury in hippocampal neurons.

### EXAMPLE 5

### In vitro cortical neuron repair by the Isopropyl-D-glucopyranoside derivative Byzantionoside B

Removed the brain of E18 rat embryos and dissected into cortex and hippocampus. Subsequently, the cortex and hippocampus were separated into cortical neurons and hippocampal neurons, respectively. Then, seeded cortical neurons on 48-well plates. The first day that neurons are cultured *in vitro* is defined as day *in vitro* 0 (DIVO). On DIV2, Cytosine beta-D-arabinoside (AraC) was added to neurons to inhibit the proliferation of glial cells. On DIV8, neurons were injured using pipette tips (p10 tips) and 1.34 µM to 26.84 µM Byzantionoside B was added. ddH₂O served as the solvent control group, and the results in the figures were standardized against the 0.1% DMSO group. After 72 hours, immunostaining was performed. TUJ1 antibody was used to label neuronal cells to observe the regrowth of neurites. Scale bar = 100 µm. Images were taken using Observer Z1 microscope.

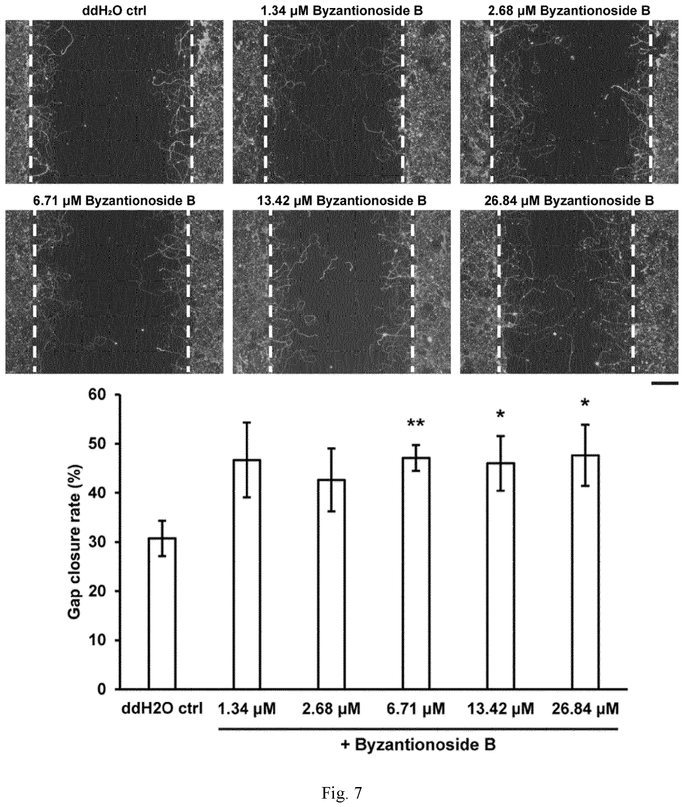

As shown in Figure 7, the degree of neuronal axon regeneration was quantified using the gap closure rate. The results of this experiment indicate that Byzantionoside B effectively promotes neurite outgrowth in cortical neurons.

### EXAMPLE 6

### In vitro hippocampal neuron repair experiment of Isopropyl-D-glucopyranoside derivative Roseoside

Removed the brain of E18 rat embryos and dissected into cortex and hippocampus. Subsequently, the cortex and hippocampus were separated into cortical neurons and hippocampal neurons, respectively. Then, seeded hippocampal neurons on 48-well plates. The first day that neurons are cultured *in vitro* is defined as day *in vitro* 0 (DIVO). On DIV2, Cytosine beta-D-arabinoside (AraC) was added to neurons to inhibit the proliferation of glial cells. On DIV8, neurons were injured using pipette tips (p10 tips) and 0.013 µM to 1294 µM Roseoside was added. ddH₂O served as the solvent control group, and the results in the figures were standardized against the 0.1% DMSO group. After 72 hours, immunostaining was performed. TUJ1 antibody was used to label neuronal cells to observe the regrowth of neurites. Scale bar = 100 µm. Images were taken using Observer Z1 microscope.

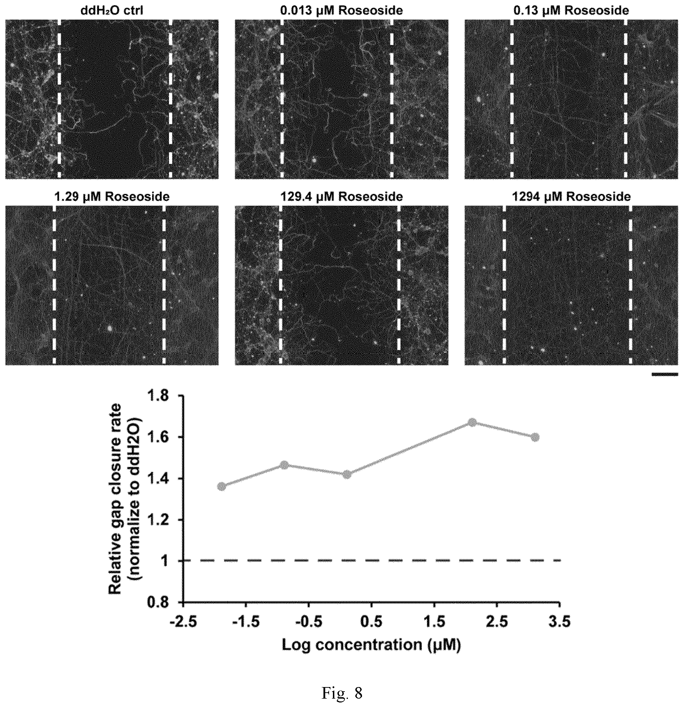

As shown in Figures 8, quantifying the degree of axonal regeneration through the gap closure rate, the immunofluorescence image with two dashed lines representing the injury area. The result indicates that Roseoside promotes neurite regrowth after injury in hippocampal neurons.

### EXAMPLE 7

### In vitro cortical neuron repair experiment of Isopropyl-D-glucopyranoside derivative Roseoside

Removed the brain of E18 rat embryos and dissected into cortex and hippocampus. Subsequently, the cortex and hippocampus were separated into cortical neurons and hippocampal neurons, respectively. Then, seeded cortical neurons on 48-well plates. The first day that neurons are cultured *in vitro* is defined as day *in vitro* 0 (DIVO). On DIV2, Cytosine beta-D-arabinoside (AraC) was added to neurons to inhibit the proliferation of glial cells. On DIV8, neurons were injured using pipette tips (p10 tips) and 1.29 µM to 25.88 µM Roseoside was added. ddH₂O served as the solvent control group, and the results in the figures were standardized against the 0.1% DMSO group. After 72 hours, immunostaining was performed. TUJ1 antibody was used to label neuronal cells to observe the regrowth of neurites. Scale bar = 100 µm. Images were taken using Observer Z1 microscope.

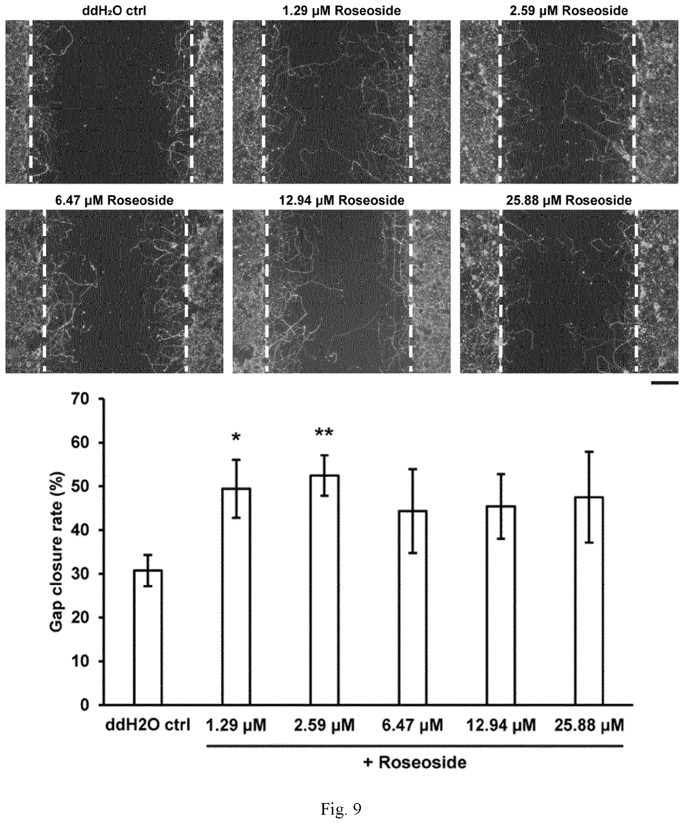

As shown in Figure 9, the degree of neuronal axon regeneration was quantified using the gap closure rate. The results of this experiment indicate that Roseoside effectively promotes dendritic outgrowth in cortical neurons

### EXAMPLE 8

### The ex vivo 3D brain slice culture of Isopropyl-D-glucopyranoside derivative Ampelopsisionoside

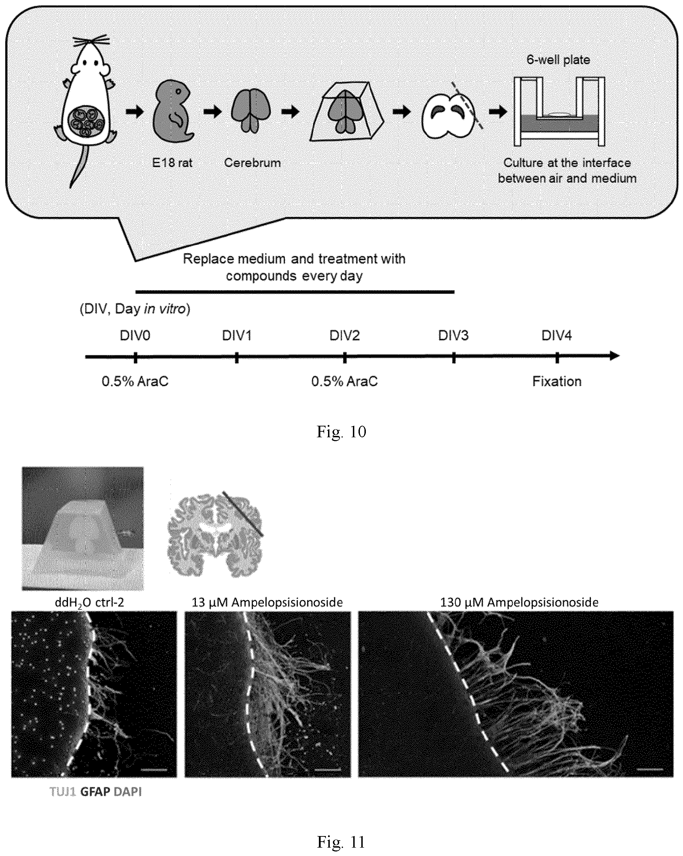

The experimental procedure is depicted in Figure 10. Took out the brain of rat embryos and embedded in low-melting agarose. The brains were sectioned using Leica microtome VT100. The tissue sections were collected with 350 µm thick and injured using a scalpel. ddH₂O or Ampelopsisionside was added to injured brain sections every day. After 96 hours, immunostaining was performed. TUJ1 antibody was used to label neuron cells. GFAP antibody was used to label glial cells. DAPI was used to label nucleus. Scale bar = 100 µm. Images were taken using Zeiss LSM800 confocal microscope.

As shown in Figure 11, the white dashed line indicated the injury site caused by a scalpel. The regenerated neurites grow from injury site toward the right side of the images. The result shows that Ampelopsisionside promotes neurite outgrowth.

### EXAMPLE 9

### The ex vivo 3D brain slice culture of Isopropyl-D-glucopyranoside derivative Byzantionoside B

The experimental procedure is depicted in Figure 10. Took out the brain of rat embryos and embedded in low-melting agarose. The brains were sectioned using Leica microtome VT100. The tissue sections were collected with 350 µm thick and injured using a scalpel. ddH₂O or Byzantionoside B was added to injured brain sections every day. After 96 hours, immunostaining was performed. TUJ1 antibody was used to label neuron cells. GFAP antibody was used to label glial cells. DAPI was used to label nucleus. Scale bar = 100 µm. Images were taken using Zeiss LSM800 confocal microscope.

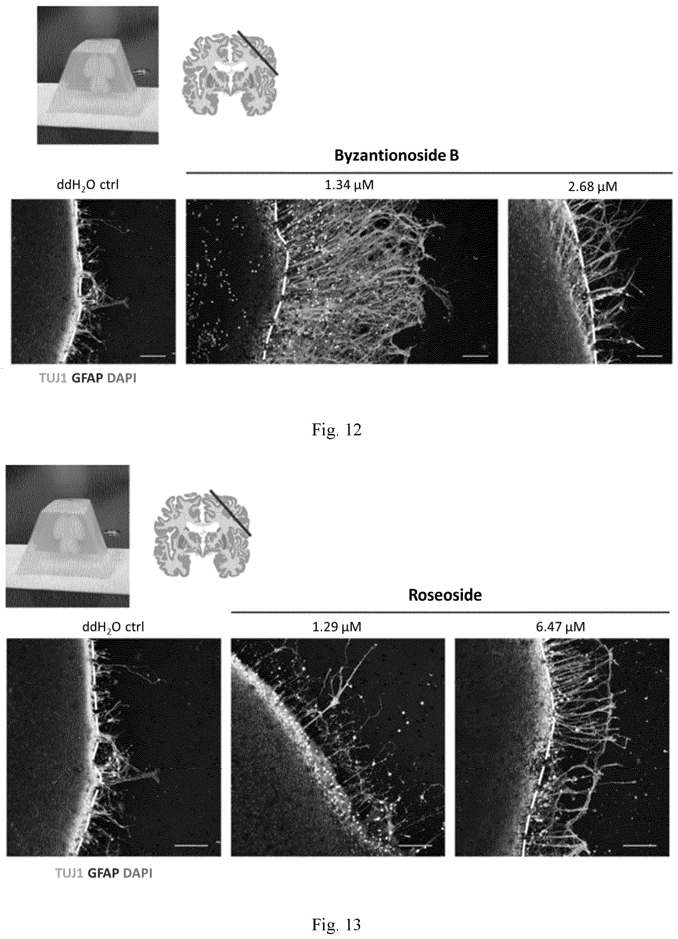

As shown in Figure 12, the white dashed line indicated the injury site caused by a scalpel. The regenerated neurites grow from injury site toward the right side of the images. The result shows that Byzantionoside B promotes neurite outgrowth.

### EXAMPLE 10

### The ex vivo 3D brain slice culture of Isopropyl-D-glucopyranoside derivative Roseoside

The experimental procedure is depicted in Figure 10. Took out the brain of rat embryos and embedded in low-melting agarose. The brains were sectioned using Leica microtome VT100. The tissue sections were collected with 350 µm thick and injured using a scalpel. ddH₂O or Roseoside was added to injured brain sections every day. After 96 hours, immunostaining was performed. TUJ1 antibody was used to label neuron cells. GFAP antibody was used to label glial cells. DAPI was used to label nucleus. Scale bar = 100 µm. Images were taken using Zeiss LSM800 confocal microscope.

As shown in Figure 13, the white dashed line indicated the injury site caused by a scalpel. The regenerated neurites grow from injury site toward the right side of the images. The result shows that Roseoside promotes neurite outgrowth.

### EXAMPLE 11

### The experiment of Isopropyl-D-glucopyranoside derivative Ampelopsisionoside promoting the recovery of motor function in mice after brain injury

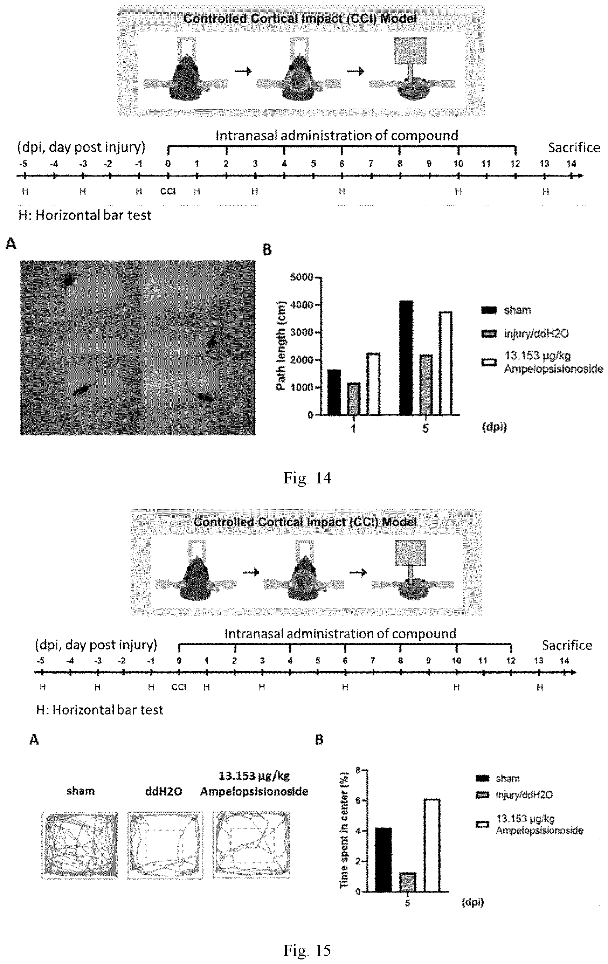

Measurement of locomotor activity via open field test on sham, injury mice. After brain injury, mice were administered 14 µg/kg of Ampelopsisionoside daily for one or five days (1 day post injury, 1 Dpi; 5 days post injury, 5 Dpi) to compare motor function. The experimental model is depicted in Figure 14, where mice were subjected to controlled cortical impact model at 0 Dpi and administered 14 µg/kg of Ampelopsisionoside via intranasal delivery at 0, 2, 4, 6, 8, 10, and 12 Dpi.

As shown in Figure 14, in Part A of this experiment, mice were placed in segmented white large acrylic box divided into four equal sections (independent open fields) and allowed to freely explore for five minutes. During this period, the movement of mice was recorded using a Microsoft LifeCam Cinema camera and analyzed using Anubis track tracking software.

As shown in Figure 14, in Part B of this experiment reveals that the length of movement path for the Ampelopsisionoside-treated group is nearly identical to that of the uninjured group, whereas the untreated brain injury group exhibits only half the path length. This indicates that the Isopropyl-D-glucopyranoside derivative Ampelopsisionoside provided by the present invention has a beneficial effect on promoting the recovery of motor function in mice after brain injury.

### EXAMPLE 12

### The experiment of Isopropyl-D-glucopyranoside derivative Ampelopsisionoside promoting the recovery of exploratory ability in mice after brain injury.

Zone exploratory activity via open field test on sham, injury, and vehicle administration mice. After brain injury, mice were administered 14 µg/kg of Ampelopsisionoside for five days (5 days post injury, 5 Dpi) to assess the exploratory ability in the area and the time spent in the central open area. The experimental model is depicted in Figure 15, where mice were subjected to controlled cortical impact model at 0 Dpi and administered 14 µg/kg of Ampelopsisionoside via intranasal delivery at 0, 2, 4, 6, 8, 10, and 12 Dpi.

As shown in Figure 15, in Part A of this experiment mice were placed in segmented white large acrylic box divided into four equal sections (independent open fields) and allowed to freely explore for five minutes. During this period, the movement of mice is recorded using a Microsoft LifeCam Cinema camera and analyzed using Anubis track tracking software.

As shown in Figure 15, in Part B of this experiment reveals that the Ampelopsisionoside-treated group and the uninjured group spend a longer time in the central area, whereas the untreated brain injury group shows minimal activity in the center. Combined with Figure 15, it can be observed that the Isopropyl-D-glucopyranoside derivative Ampelopsisionoside provided by the present invention has a beneficial effect on promoting the recovery of exploratory ability in mice after brain injury.

### EXAMPLE 13

### The experiment of Isopropyl-D-glucopyranoside derivative Ampelopsisionoside promoting the motor coordination in mice after brain injury

Using the horizontal bar test, mice were placed on brass rods positioned at a height of 49 cm above the ground, with lengths of 38 cm and diameters of 2 mm, 4 mm, and 6 mm. Mice were required to grasp the brass rod with their forepaws, and their ability to move and coordinate was assessed by measuring the time spent on the brass rod and whether they reached the platform at the end of the rod. Evaluation criteria were as follows: 1 point for 1-5 seconds, 2 points for 5-10 seconds, 3 points for 10-20 seconds, 4 points for 20-30 seconds, and 5 points for over 30 seconds or reaching the platform.

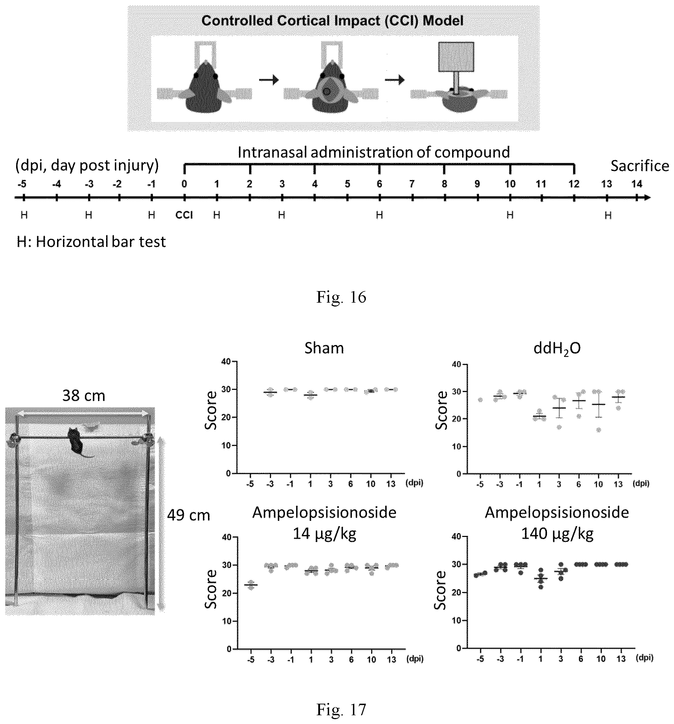

The experimental model is depicted in Figure 16, involved pre-training at -5, -3, and -1 days post injury (Dpi) (5, 3, and 1 day(s) before brain injury). At 0 Dpi, the controlled cortical impact model (CCI model) was used to induce brain injury in mice. Subsequently, at 0, 2, 4, 6, 8, 10, and 12 Dpi, mice were administered Ampelopsisionoside at doses of 14 or 140 µg/kg via intranasal delivery. Horizontal bar experiments were conducted at 1, 3, 6, 10, and 13 Dpi.

As shown in Figure 17, it is evident that the group receiving only water (ddH₂O) after brain injury exhibited a loss of motor coordination. However, in the groups receiving Ampelopsisionoside, motor coordination was similar to that of the Sham (surgery control) group, indicating a significant promotion of motor coordination recovery.

### EXAMPLE 14

### Isopropyl-D-glucopyranoside derivative Byzantionoside B promotes ex vivo retinal neuronal tissue repair

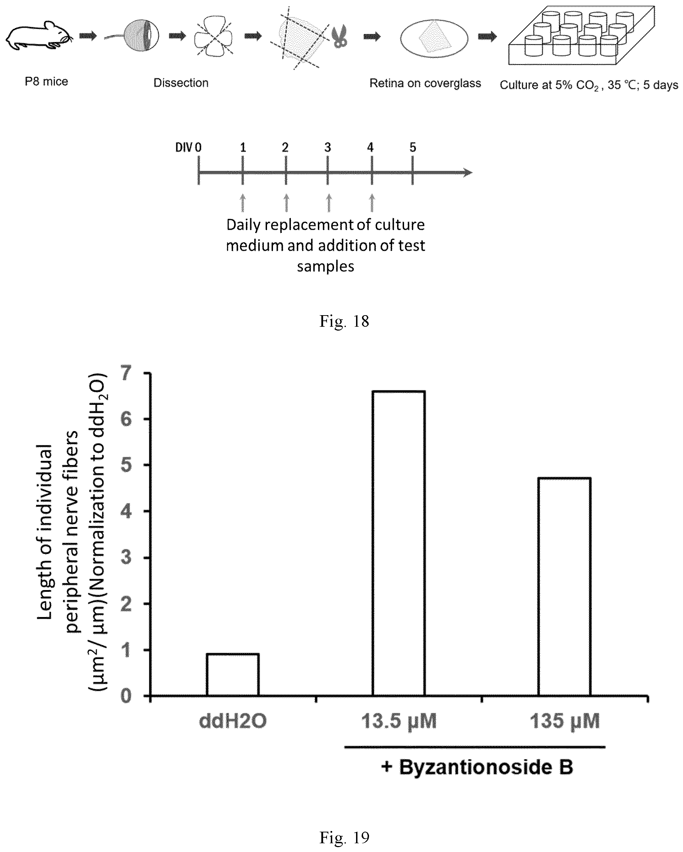

The experimental design and procedure are showed in Figure 18. Retinal explants were obtained from C57BL/6 mice at postnatal day 8. Following the sacrifice of mice, the eyes were enucleated, and the eyeballs were dissected using forceps and microscissors to separate the retina and remove the vitreous. Subsequently, the dissected retinas were quartered and trimmed along the edges of the tissue using microscissors. Each quarter was cultured on an 18 mm round coverslip and placed in a 12-well plate inside a CO₂ incubator at 35°C for five days. The culture medium was replaced daily with fresh medium containing either 13.5 µM or 135 µM Byzantionoside B. On the fifth day of culture, the retinal tissues were fixed with a mixture of 0.1% glutaraldehyde solution and 4% paraformaldehyde at room temperature for one hour. Immunostaining was performed using primary antibodies against the axonal marker beta-III-tubulin (TUJ1) and DAPI to label neurons and cell nuclei, respectively. Images were acquired using a super-resolution upright confocal microscope (LSM-800, Carl Zeiss). ImageJ software was utilized for image analysis, wherein the tissue boundary was delineated to calculate the perimeter and the area of neuronal fibers outside the tissue boundary. The ratio of neuronal fiber area to tissue boundary perimeter was then calculated to determine the unit perimeter neuronal fiber length.

As shown in Figure 19, the group treated with Byzantionoside B exhibited significant recovery of injured retinal neurons compared to the group treated with water (ddH₂O) alone.

### EXAMPLE 15

### Isopropyl-D-glucopyranoside derivative Roseoside promotes ex vivo retinal neuronal tissue repair

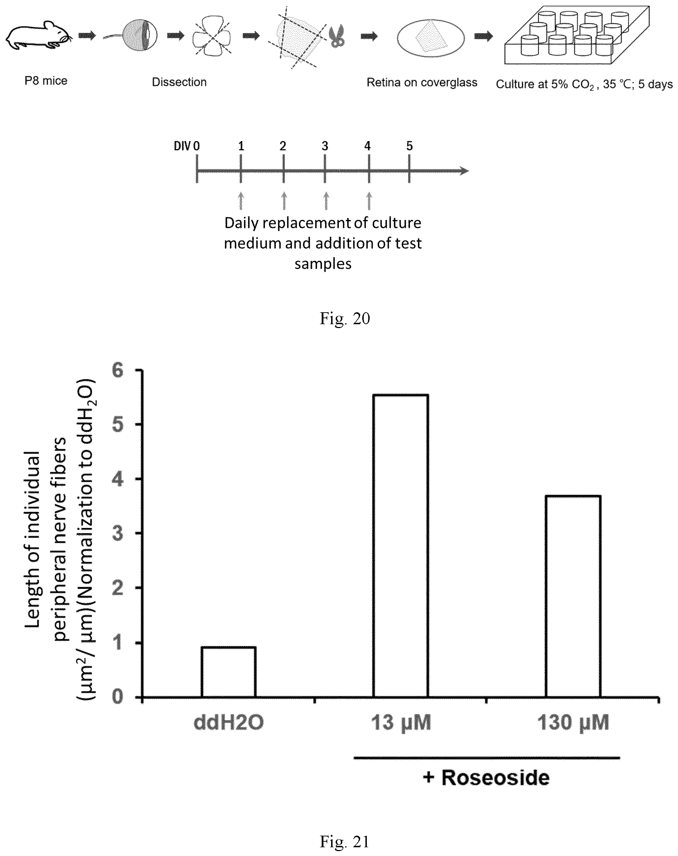

The experimental design and procedure are showed in Figure 20. Retinal explants were obtained from C57BL/6 mice at postnatal day 8. Following the sacrifice of mice, the eyes were enucleated, and the eyeballs were dissected using forceps and microscissors to separate the retina and remove the vitreous. Subsequently, the dissected retinas were quartered and trimmed along the edges of the tissue using microscissors. Each quarter was cultured on an 18 mm round coverslip and placed in a 12-well plate inside a CO₂ incubator at 35°C for five days. The culture medium was replaced daily with fresh medium containing either 13 µM or 130 µM Roseoside. On the fifth day of culture, the retinal tissues were fixed with a mixture of 0.1% glutaraldehyde solution and 4% paraformaldehyde at room temperature for one hour. Immunostaining was performed using primary antibodies against the axonal marker beta-III-tubulin (TUJ1) and DAPI to label neurons and cell nuclei, respectively. Images were acquired using a super-resolution upright confocal microscope (LSM-800, Carl Zeiss). Imaged software was utilized for image analysis, wherein the tissue boundary was delineated to calculate the perimeter and the area of neuronal fibers outside the tissue boundary. The ratio of neuronal fiber area to tissue boundary perimeter was then calculated to determine the unit perimeter neuronal fiber length.

As shown in Figure 21, the group treated with Roseoside exhibited significant recovery of injured retinal neurons compared to the group treated with water (ddH₂O) alone.

### EXAMPLE 16

### The consecutive synthesis steps of Isopropyl-D-glucopyranoside derivatives

The continuous synthesis steps of Isopropyl-D-glucopyranoside derivatives in this invention are as follows:

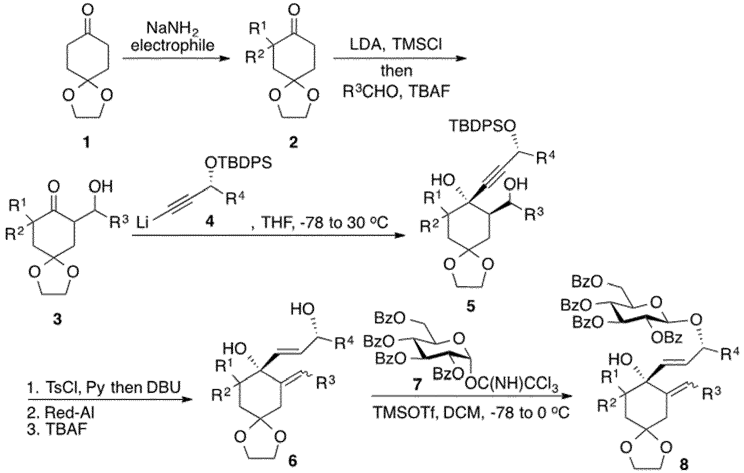

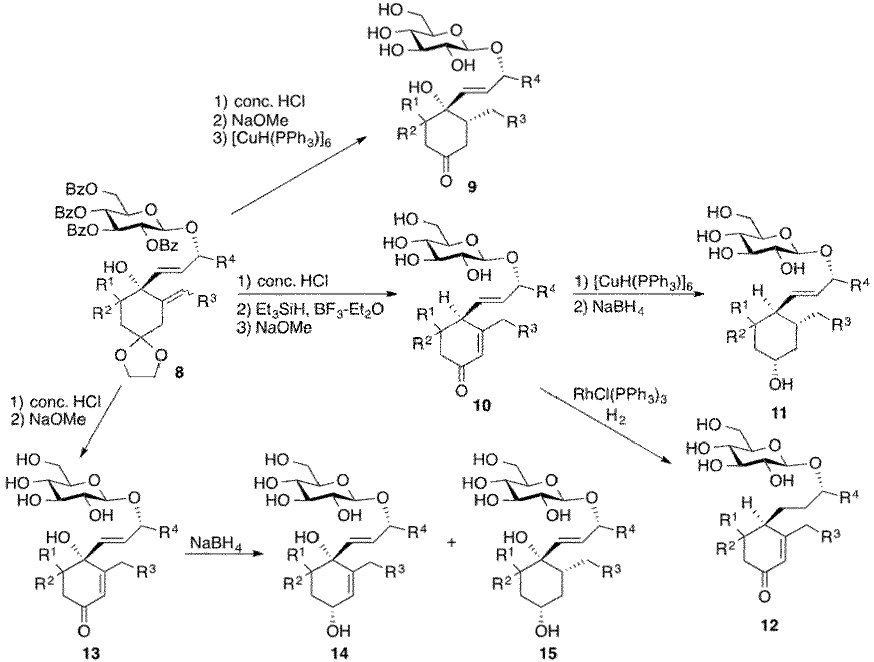

All examples provided herein are intended for pedagogical purposes of aiding the reader in understanding the invention and the concepts contributed by the inventors to further the art, and are not to be construed as limitations to such specifically recited examples and conditions, nor does the organization of such examples in the specification relate to a showing of the superiority or inferiority of the invention. Although one or more embodiments of the present invention have been described in detail, it should be understood that the various changes, substitutions, and alterations could be made hereto without departing from the spirit and scope of the invention.

It is intended that the specification and examples be considered as examples only, with a true scope and spirit of the invention being indicated by the following claims.

## Claims

1. An Isopropyl-D-glucopyranoside derivative, wherein the Isopropyl-D-glucopyranoside derivative has the structure of Formula (1), 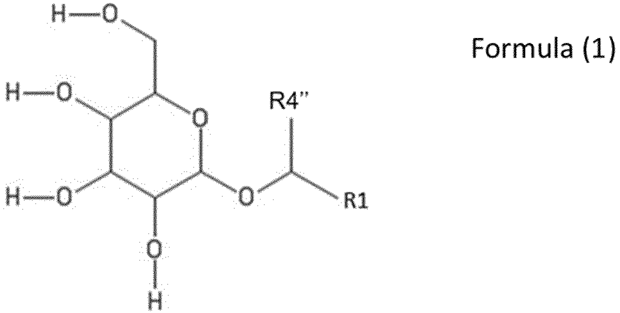 wherein R1 can is substituted or nonsubstituted Formula (2) or Formula (3), 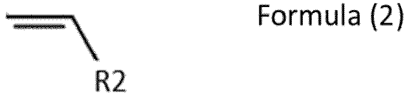 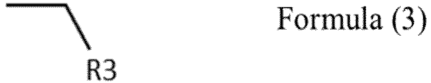 wherein R2 and R3 is substituted or nonsubstituted Formula (4) or Formula (5), 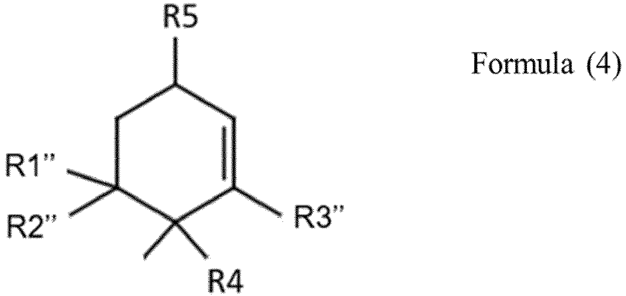 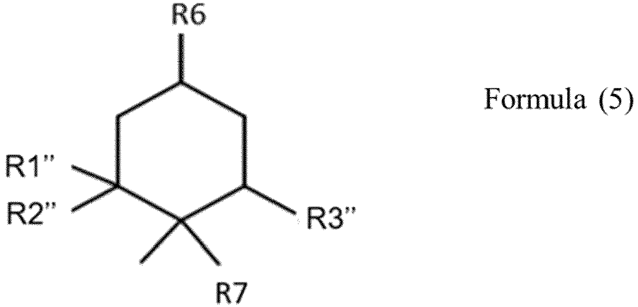
wherein R4 is hydrogen, deuterium, tritium, hydroxyl, or halogen,
wherein R5 is hydrogen, deuterium, tritium, hydroxyl, carbonyl, or halogen,
wherein R6 is hydrogen, deuterium, tritium, hydroxyl, carbonyl, or halogen,
wherein R7 is hydrogen, deuterium, tritium, hydroxyl, carbonyl, or halogen,
wherein R1" is Methyl, ethyl, propyl, hydrogen, deuterium, tritium, or hydroxyl,
wherein R2" is Methyl, ethyl, propyl, hydrogen, deuterium, tritium, or hydroxyl,
wherein R3" is Methyl, ethyl, propyl, hydrogen, deuterium, or tritium.,
wherein R4" is Methyl, ethyl, propyl, hydrogen, deuterium, or tritium.

2. The Isopropyl-D-glucopyranoside derivative of claim 1, wherein when R1 is Formula (2), 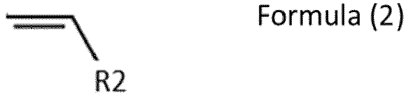 R2 and R3 is substituted or nonsubstituted Formula (4) or Formula (5), 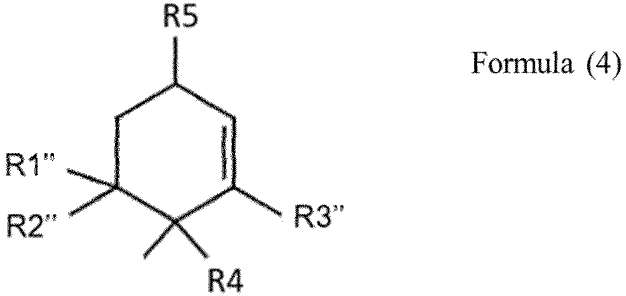 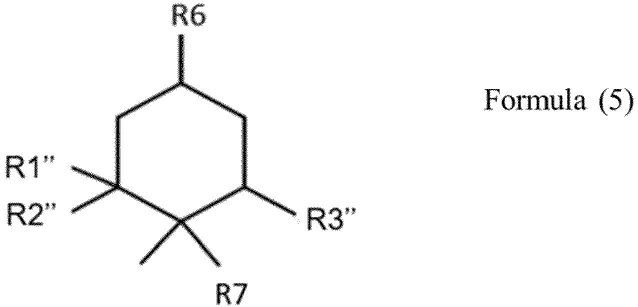
wherein R4 is hydrogen, deuterium, tritium, hydroxyl, or halogen,
wherein R5 is hydrogen, deuterium, tritium, hydroxyl, carbonyl, or halogen,
wherein R6 is hydrogen, deuterium, tritium, hydroxyl, carbonyl, or halogen,
wherein R7 is hydrogen, deuterium, tritium, hydroxyl, carbonyl, or halogen,
wherein R1" is Methyl, ethyl, propyl, hydrogen, deuterium, tritium, or hydroxyl,
wherein R2" is Methyl, ethyl, propyl, hydrogen, deuterium, tritium, or hydroxyl,
wherein R3" is Methyl, ethyl, propyl, hydrogen, deuterium, or tritium.,
wherein R4" is Methyl, ethyl, propyl, hydrogen, deuterium, or tritium.

3. The Isopropyl-D-glucopyranoside derivative of claim 1, wherein when R1 is Formula (3), 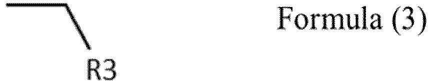 R2 and R3 is substituted or nonsubstituted Formula (4) or Formula (5), 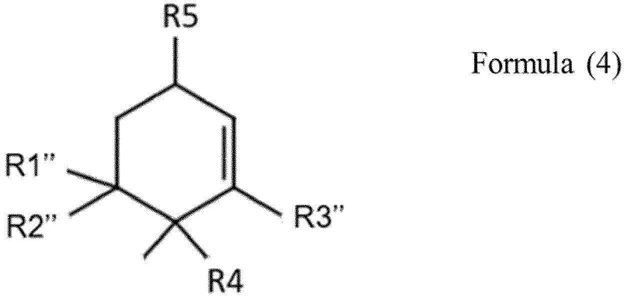 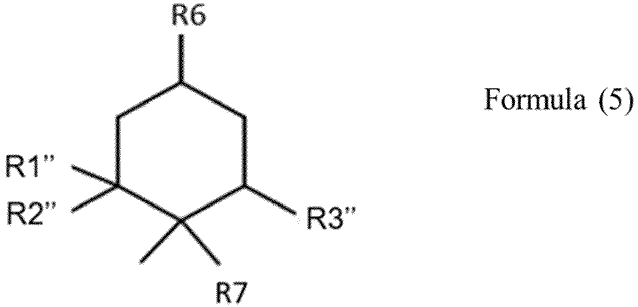
wherein R4 is hydrogen, deuterium, tritium, hydroxyl, or halogen,
wherein R5 is hydrogen, deuterium, tritium, hydroxyl, carbonyl, or halogen,
wherein R6 is hydrogen, deuterium, tritium, hydroxyl, carbonyl, or halogen,
wherein R7 is hydrogen, deuterium, tritium, hydroxyl, carbonyl, or halogen,
wherein R1" is Methyl, ethyl, propyl, hydrogen, deuterium, tritium, or hydroxyl,
wherein R2" is Methyl, ethyl, propyl, hydrogen, deuterium, tritium, or hydroxyl,
wherein R3" is Methyl, ethyl, propyl, hydrogen, deuterium, or tritium.,
wherein R4" is Methyl, ethyl, propyl, hydrogen, deuterium, or tritium.

4. The Isopropyl-D-glucopyranoside derivative of claim 1, wherein the Isopropyl-D-glucopyranoside derivative comprises following compounds: 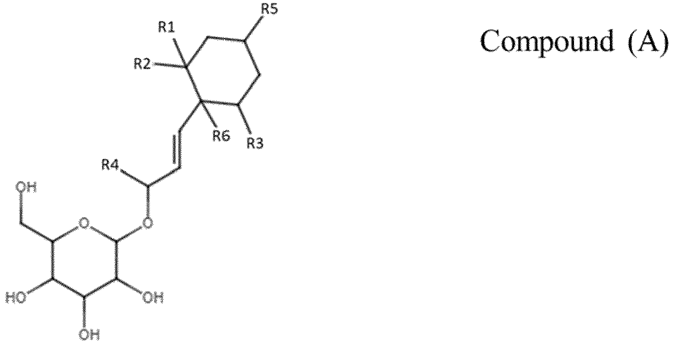 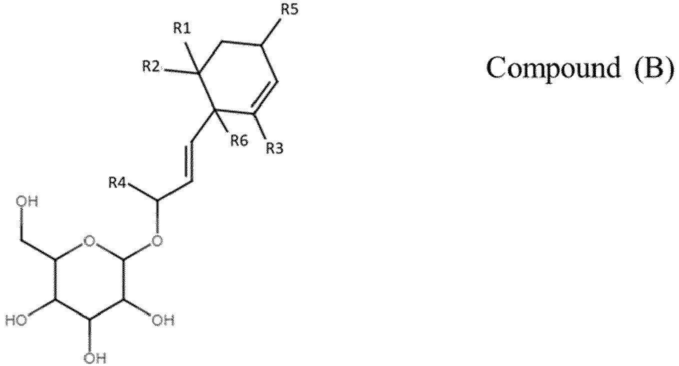 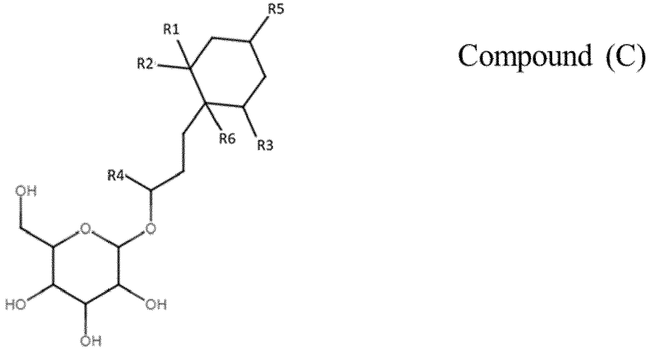 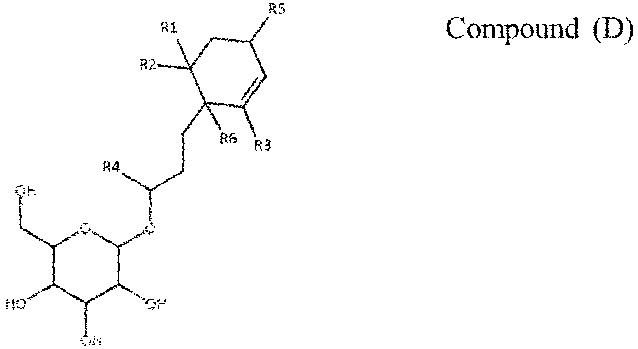
wherein R1 is methyl, ethyl, propyl, hydrogen, deuterium, tritium, or hydroxyl,
wherein R2 is methyl, ethyl, propyl, hydrogen, deuterium, tritium, or hydroxyl,
wherein R4 is methyl, ethyl, propyl, hydrogen, deuterium, or tritium,
wherein R4 is methyl, ethyl, propyl, hydrogen, deuterium, or tritium,
wherein R5 is hydrogen, deuterium, tritium, hydroxyl, carbonyl, or halogen,
wherein R6 is hydrogen, deuterium, tritium, hydroxyl, or halogen.

5. A method for preparing an Isopropyl-D-glucopyranoside derivative of Formula (6), wherein the method comprises:
(A) Transforming Formula (7) into Formula (8) of the following structure by including a dialkylation, an acylation, and an acetylide addition; 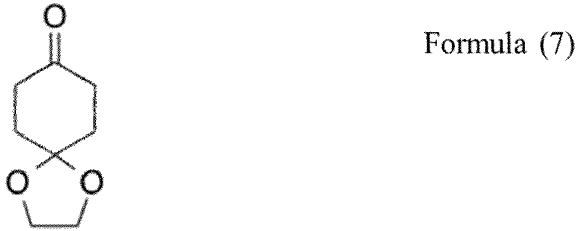 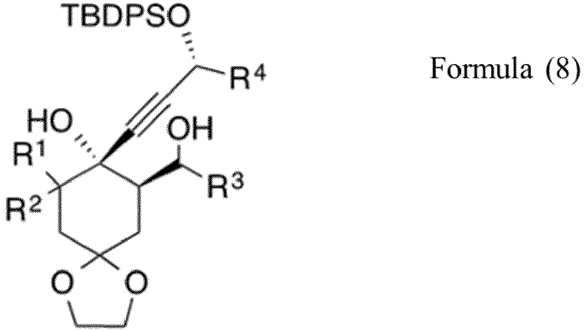
wherein R1 is Me, Et, Pr, H, or OH; R2 = Me, Et, Pr, H, or OH; R3 = Me, Et, Pr, or H; R4 = Me, Et, Pr or H;
wherein in the dialkylation, the compound of Formula (7) is carried out with an electrophilic reagent 1, which can be MeI, EtI, MeOTf, EtOTf, PrI, PrBr/NaI, or Me₂SO₄;
wherein in the acylation, the reagent can be HCHO, MeCHO, or EtCHO;
wherein this intermediate undergoes further anionic nucleophilic addition reaction with an alkyne, leading to the formation of compound (8), wherein the alkyne nucleophilic addition reagent comprises an alkyne bearing a functional group R4, along with a base 1, which includes n-butyllithium (nBuLi), lithium diisopropylamide (LDA), or lithium bis(trimethylsilyl)amide (LHMDS), wherein the functional group R4 can be methyl, ethyl, propyl, hydrogen, deuterium, or tritium;
(B) Transforming compound of Formula (8) into compound of Formula (9) by including an olefination, a reduction, and a deprotection; 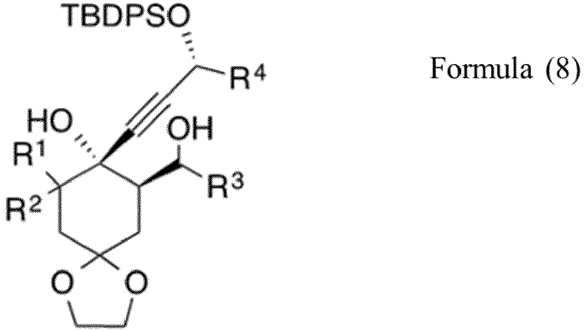 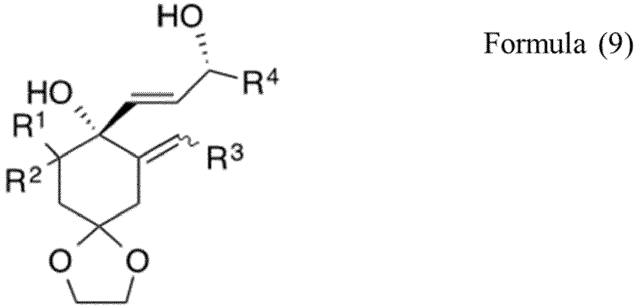
wherein the compound of Formula (8) in the olefination is first performed with an electrophilic reagent 2 and an amine base reagent at a reduced temperature to form the compound of Formula (9), wherein the electrophilic reagent 2 can be mesyl chloride, tosyl chloride, acetic anhydride, benzoic anhydride, methyl iodide, and dimethyl sulfate,
wherein the amine base reagent can be triethyl amine, diethyl amine, pyridine, pyrrolidine, ethyldisiopropyl amine, 2,6-lutidine, and 1,4-diazabicyclo[2,2,2]octane, wherein the reduced temperature is -30 to 25°C, after that a base 2 is then added to the above reaction mixture to complete the olefination, wherein the base 2 can be potassium tert-butoxide, sodium hydroxide, sodium methoxide, or 1,8-diazabicyclo[5,4,0]undec-7-ene, following completion, the reduction reaction is carried out with a reducing agent 1 as the reducing agent, wherein the reducing agent 1 can be LiAlH₄, diisobutyl aluminum hydride (DIBAL), NaBH₄, NaB(OAc)₃H, Lithium triethylborohydride, or sodium bis(2-methoxyethoxy)luminiumhydride, the reaction temperature of the reduction is -50 to 25°C;
wherein following completion, the deprotection is carried out with a desilylation reagent 1 to conduct the deprotection reaction to form formula (9), wherein the deprotection reagent 1 is tetrabutyl ammonium fluoride (TBAF), HF-py, HCl, or potassium tert-butoxide, wherein the reaction temperature of the deprotection is -30 to 25 °C;
(C) Reacting Formula (9) with Formula (10) by glycosylation to form Formula (11); 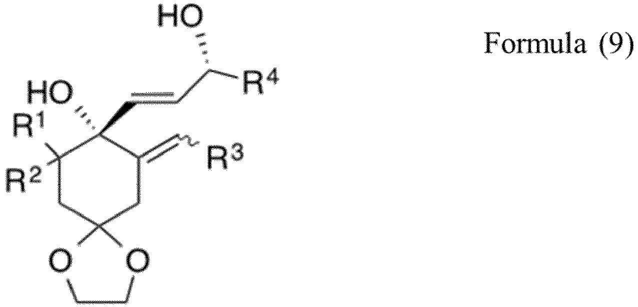 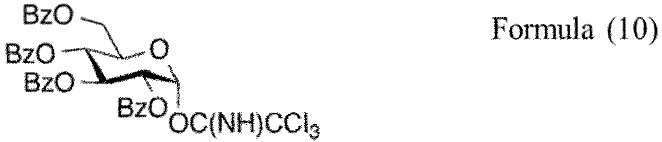 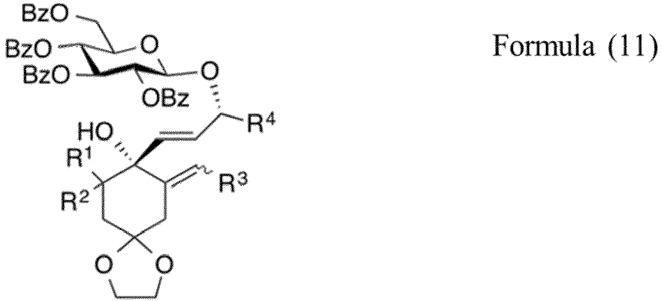 wherein the compound of Formula (9) undergoes the glycosylation reaction carried out with a glycosylating agent 1 to yield the compound of Formula (11), wherein the glycosylation reagent 1 can be NIS/AgOTf, BSP/TTBP/Tf7O, TMSOTf/NIS, TMSOTf, AgOTf, or CuOTf, wherein the reaction temperature of the deprotection is -78 to 0 °C;
(D)Transforming the compound of Formula (11) into the compound of Formula (6) by including a deprotection of acetal, an isomerization, a removal of benzoyl group protection, and a 1,4-reduction;
wherein the compound of Formula (11) undergoes the deprotection of acetal and the isomerization initially, wherein the deprotection of acetal and the isomerization are carried out with a reagent 1, wherein the reagent 1 can be AcOH(aq), HCl(aq), TFA+H₂O, TsOH+H₂O, HCl in 1,4-dioxane, or H₂SO₄ (aq);
subsequently, removal of benzoyl group is carried out using a deprotection reagent, wherein the deprotection reagent can be NaOH, NaOMe, NaOEt, KOH, KOMe, KOEt, HCl in MeOH, HCl(aq), TFA+H₂O, or H₂SO₄(aq);
following that, a 1,4-reduction is conducted to obtain the compound of Formula (6), where the 1,4-reduction is carried out with a 1,4-reduction reagent, wherein the 1,4-reduction reagent can be NaBH₄, LiAlH₄, NaB(OAc)₃H, NaBCNH₃, L-selectride, [CuH(PPh₃)]₆, diisobutyl aluminum hydride (DIBAL), or H₂/Pd/C, wherein the reaction temperature of the 1,4-reduction is -78 to 25 °C;
wherein R1 is methyl, ethyl, propyl, hydrogen, deuterium, tritium, or hydroxyl,
wherein R2 is methyl, ethyl, propyl, hydrogen, deuterium, tritium, or hydroxyl,
wherein R4 is methyl, ethyl, propyl, hydrogen, deuterium, or tritium,
wherein R4 is methyl, ethyl, propyl, hydrogen, deuterium, or tritium.

6. The method of claim 5, wherein the step (B) further can comprise following synthetic step: (B1) Converting the compound of Formula (9) by a deprotection of acetal, an isomerization, and a removal of benzoyl group to yield the compound of Formula (15); 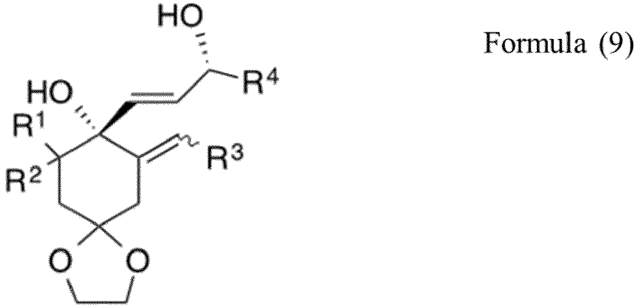 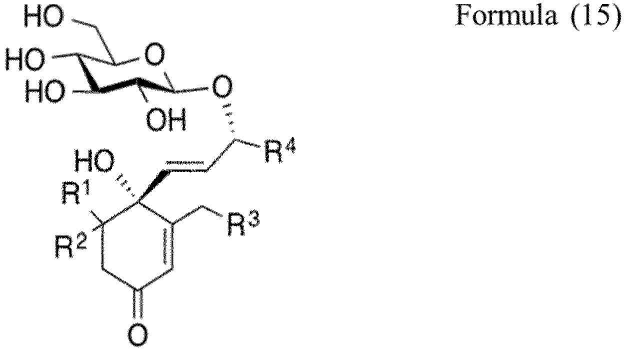
wherein the compound of Formula (9) undergoes the deprotection of acetal and the isomerization initially, wherein the deprotection of acetal and the isomerization are carried out with a deprotection of acetal and isomerization reagent, respectively, wherein the deprotection of acetal and isomerization reagent can be AcOH(aq), HCl(aq), TFA+H₂O, TsOH+H₂O, HCl in 1,4-dioxane, or H₂SO₄(aq);
subsequently, removal of benzoyl group is carried out using a deprotection reagent, wherein the deprotection reagent can be NaOH, NaOMe, NaOEt, KOH, KOMe, KOEt, HCl in MeOH, HCl(aq), TFA+H₂O, or HCl (aq);
wherein R1 is methyl, ethyl, propyl, hydrogen, deuterium, tritium, or hydroxyl,
wherein R2 is methyl, ethyl, propyl, hydrogen, deuterium, tritium, or hydroxyl,
wherein R4 is methyl, ethyl, propyl, hydrogen, deuterium, or tritium,
wherein R4 is methyl, ethyl, propyl, hydrogen, deuterium, or tritium.

7. The method of claim 6, after the step (B 1) further can comprise following synthetic step: (B2) Transforming the compound of Formula (15) into the compounds of Formulas (16) and Formulas (17) by a selective reduction reaction; 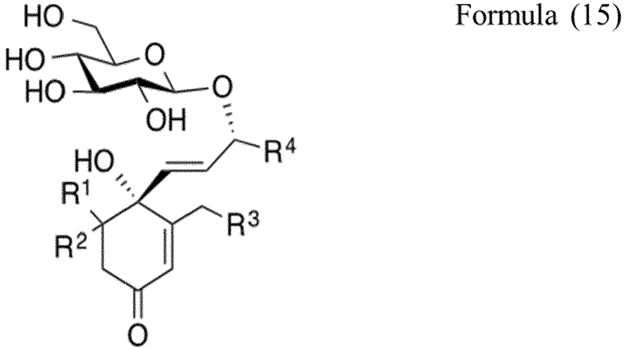 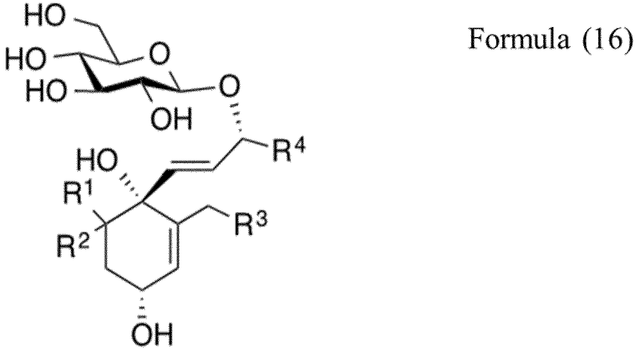 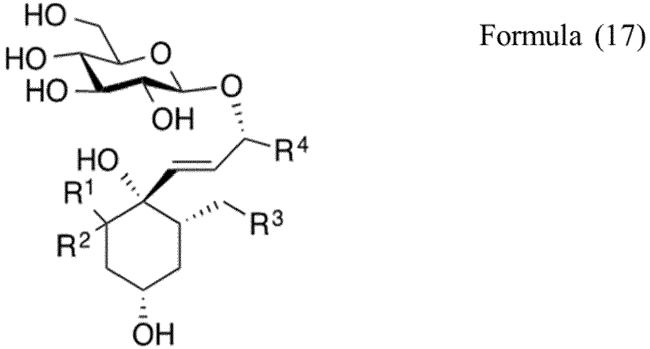
wherein the compound of Formula (15) is subjected to selective reduction carried out with a selective reduction reagent to obtain the compounds of formulas (16) and (17), wherein the selective reduction reagent can be NaBH₄, LiAlH₄, NaB(OAc)₃H, NaBCNH₃, L-selectride, [CuH(PPh₃)]₆, diisobutyl aluminum hydride (DIBAL) or H₂/Pd/C, wherein the reaction temperature of the selective reduction is -78 to 25 °C;
wherein R1 is methyl, ethyl, propyl, hydrogen, deuterium, tritium, or hydroxyl,
wherein R2 is methyl, ethyl, propyl, hydrogen, deuterium, tritium, or hydroxyl,
wherein R4 is methyl, ethyl, propyl, hydrogen, deuterium, or tritium,
wherein R4 is methyl, ethyl, propyl, hydrogen, deuterium, or tritium.

8. A method for preparing an Isopropyl-D-glucopyranoside derivative of Formula (12), wherein the method comprises: 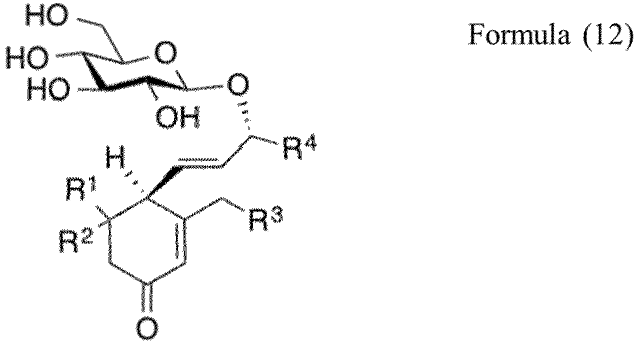
(A) Transforming the compound of Formula (11) into the compounds of Formulas (12) by a conversion reaction; 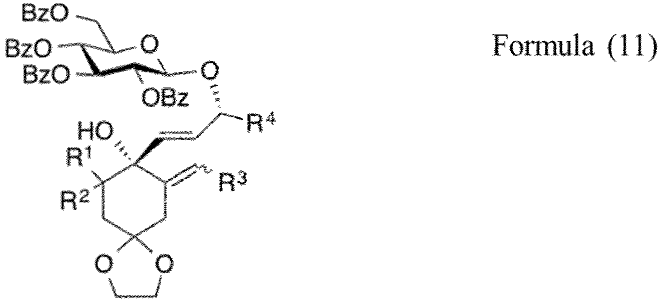 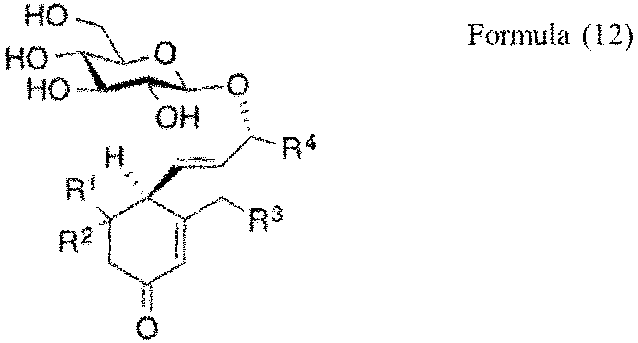
wherein the conversion reaction comprises a deprotection of acetal, an isomerization, a stereoselective deoxygenation, and a removal of benzoyl protection, wherein the deprotection of acetal and the isomerization is carried out with an acidic reagent, wherein the acidic reagent can be AcOH(aq), HCl(aq), TFA+H₂O, TsOH+H₂O, HCl in 1,4-dioxane, or H₂SO₄(aq);
following by a stereoselective deoxygenationn with a reducing reagent 2 at a reduced temperature 1, wherein the reducing reagent 2 can be NaBH₄, LiAlH₄, NaB(OAc)₃H, NaBCNH₃, Et₃SiH/BF₃-Et₂O, L-selectride, [CuH(PPh₃)]₆, or diisobutyl aluminum hydride (DIBAL), wherein the amount of the reducing reagent can be 0.5, 1.0, 2.0, 3.0, 4.0, or 5.0 equivalences, wherein the reduced temperature 1 in the stereoselective deoxygenation is -78 to 25 °C;
after that the intermediate is then subjected to removal of benzoyl group with a deprotection reagent 2 to produce the compound of the Formula (12), wherein the deprotection reagent 2 can be NaOH, NaOMe, NaOEt, KOH, KOMe, KOEt, HCl in MeOH, HCl(aq), TFA+H₂O, or H₂SO₄(aq);
wherein R1 is methyl, ethyl, propyl, hydrogen, deuterium, tritium, or hydroxyl,
wherein R2 is methyl, ethyl, propyl, hydrogen, deuterium, tritium, or hydroxyl,
wherein R4 is methyl, ethyl, propyl, hydrogen, deuterium, or tritium,
wherein R4 is methyl, ethyl, propyl, hydrogen, deuterium, or tritium.

9. The method of claim 8, after the step (A) further can comprise following synthetic step: (B) Transforming the compound of Formula (12) into the compound of Formula (13) through a conversion reaction, 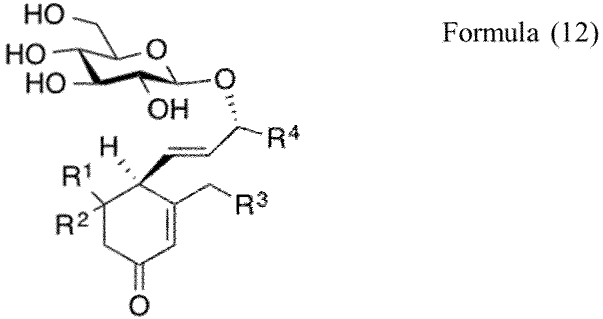 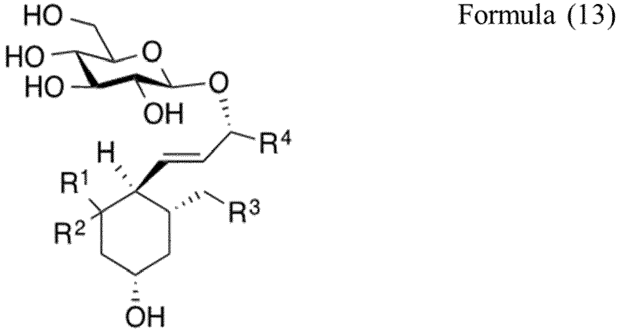
wherein the conversion reaction comprises a regio- and stereo-selective 1,4-reduction/carbonyl reduction; wherein the region- and stereo-selective 1,4-reduction is performed with a reducing reagent 3 at a reduced temperature 2, wherein the reducing reagent 3 can be NaBH₄, LiAlH₄, NaB(OAc)₃H, NaBCNH₃, L-selectride, [CuH(PPh₃)]₆, diisobutyl aluminum hydride (DIBAL) or H₂/Pd/C, wherein the amount of the reducing reagent 3 can be 0.5, 1.0, 2.0, 3.0, 4.0, or 5.0 equivalences, wherein the reduced temperature 2 in the regio- and stereo-selective 1,4-reduction/carbonyl reduction is -78 to 25 °C;
wherein R1 is methyl, ethyl, propyl, hydrogen, deuterium, tritium, or hydroxyl,
wherein R2 is methyl, ethyl, propyl, hydrogen, deuterium, tritium, or hydroxyl,
wherein R4 is methyl, ethyl, propyl, hydrogen, deuterium, or tritium,
wherein R4 is methyl, ethyl, propyl, hydrogen, deuterium, or tritium.

10. The method of claim 8, after the step (B) further can comprise following synthetic step: (C) Transforming the compound of Formula (12) into the compound of Formula (14) through a selective alkene reduction; 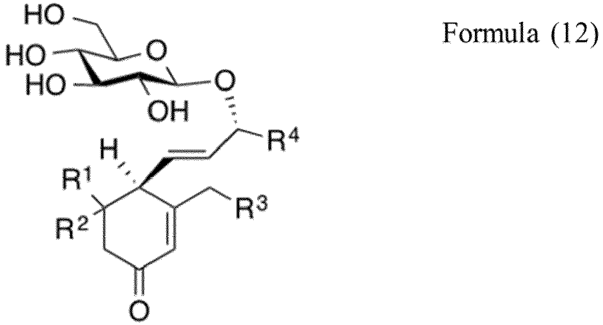 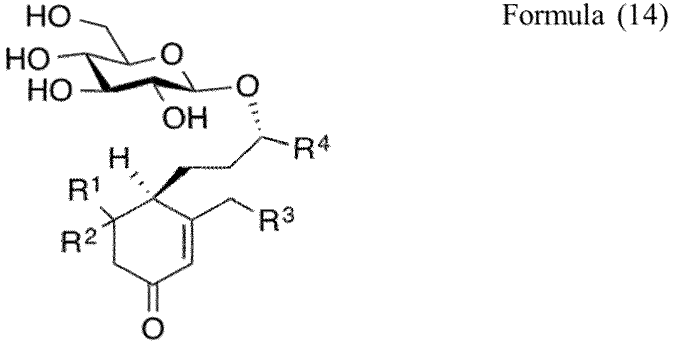
wherein the selective alkene reduction is carried out with an reducing reagent 4, wherein the reducing reagent 4 can be NaBH₄, LiAlH₄, NaB(OAc)₃H, NaBCNH₃, L-selectride, [CuH(PPh₃)]₆, diisobutyl aluminum hydride (DIBAL), H₂/Pd/C or RhCl(PPH₃)₃/H₂, wherein the reduced temperature in the selective alkene reduction is -78 to 25 °C;
wherein R1 is methyl, ethyl, propyl, hydrogen, deuterium, tritium, or hydroxyl,
wherein R2 is methyl, ethyl, propyl, hydrogen, deuterium, tritium, or hydroxyl,
wherein R4 is methyl, ethyl, propyl, hydrogen, deuterium, or tritium,
wherein R4 is methyl, ethyl, propyl, hydrogen, deuterium, or tritium.

11. A method for preparing a dug treating nerve injury, wherein the drug is the Isopropyl-d-glucopyranoside derivatives of claim 1.

12. The method of claim 11, wherein the neural injury is central nervous system or peripheral nervous system injury.

13. The method of claim 11, wherein the nerve injury is a neuron injury.

14. The method of claim 11, wherein the nerve injury comprises cortical neurons, hippocampal nerves, or retinal neurons.

15. The method of claim 1, wherein the treating is neural regeneration, increase in the number of neurons or neural repair.

16. The method of claim 1, wherein the Isopropyl-d-glucopyranoside derivatives, when administered nasally, can penetrate the blood-brain barrier to enter the brain.

17. The method of claim 1, wherein the effective dose range for Isopropyl-d-glucopyranoside derivatives is from 9.674 nM to 1342 µM.
